# EUROPÄISCHE PATENTSCHRIFT

(11) **EP 2 503 974 B1**
(45) Veröffentlichungstag und Bekanntmachung des Hinweises auf die Patenterteilung: **27.05.2015**
(21) Anmeldenummer: 10790349.4
(22) Anmeldetag: 26.11.2010
(51) Int. Cl.: A61F 13/534, A61F 13/15, A61F 13/537, B01J 20/26

(54) **ABSORBIERENDE STRUKTUR**
ABSORBENT STRUCTURE
STRUCTURE ABSORBANTE

(30) Priorität: 27.11.2009 DE 102009055951
(43) Veröffentlichungstag der Anmeldung: 03.10.2012
(73) Patentinhaber: Glatfelter Falkenhagen GmbH, 16928 Pritzwalk (DE)
(72) Erfinder: RÖTTGER, Henning, 24568 Kaltenkirchen (DE); VOLKMER, Reno, 16928 Pritzwalk OT Falkenhagen (DE)
(74) Vertreter: Von Kreisler Selting Werner - Partnerschaft von Patentanwälten und Rechtsanwälten mbB
(86) Internationale Anmeldenummer: PCT/EP2010/007186
(87) Internationale Veröffentlichungsnummer: WO 2011/063975

(56) Entgegenhaltungen:
- EP-A1- 0 600 454
- WO-A1-00/74620
- WO-A1-03/086259
- WO-A1-2005/061120
- WO-A2-2005/041815
- US-A1- 2003 234 468

## Beschreibung

Die vorliegende Erfindung betrifft eine absorbierende Struktur mit einer Flüssigkeitsaufnahmeschicht, einer Flüssigkeitsspeicherschicht und einer Flüssigkeitsverteilungsschicht unter Verwendung von Cellulosefasern und bevorzugt superabsorbierenden Polymer-Partikeln. Das superabsorbierende Polymer kann auch in anderer Form, zum Beispiel mittels eines faserartigen Gebildes vorliegen. Bevorzugt ist die absorbierende Struktur zum überwiegenden Teil mit Cellulosefasern hergestellt.

Airlaid-Produkte unter Verwendung von Cellulose und superabsorbierenden Polymer-Partikeln, abgekürzt SAP-Partikel, sind seit Jahren bekannt und werden als Lagenmaterial in Hygieneprodukten, Medikalprodukten und industriellen Produkten genutzt.

Beispielsweise wird in der WO 00/74620 eine einheitliche absorbierende Struktur unter Verwendung von Airlaid-Material, Cellulosefasern und Bindemitteln, vorzugsweise basierend auf Latex und/oder polyolefinhaltigen Bikomponentenfasern beschrieben, die eine Flüssigkeitsaufnahmeschicht, eine Flüssigkeitsspeicherschicht und eine Flüssigkeitsverteilungsschicht mit einer jeweiligen Porenstruktur aufweist, wobei die mittlere Porengröße einer jeden Schicht in einem Gradienten in Richtung von der Aufnahmeschicht zur Verteilungsschicht hin abnimmt.

Bei den bisher bekannten Produkten wird für den Transport von Flüssigkeiten ein Kapillareffekt genutzt, der durch die Wahl der Porenstruktur erzeugt wird, um die Flüssigkeit gezielt in eine Speicherlage zu führen und unerwünschte Rücknässungseffekte, die durch ein Auslaufen von Flüssigkeit aus einer Flüssigkeitsaufnahmeschicht in eine Flüssigkeitsverteilungsschicht und über diese hinaus, beispielsweise auf die Haut eines Trägers eines Hygieneprodukts auftreten können, zu vermindern.

Es wäre daher wünschenswert, ein Produkt zur Verfügung zu haben, mit welchem derartige Rücknässungseffekte vermieden werden können und gleichzeitig eine möglichst effiziente Nutzung der Absorptionsleistung von superabsorbierenden Komponten sichergestellt wird.

Es ist daher Aufgabe der vorliegenden Erfindung, ein Produkt, ein Verfahren und eine Vorrichtung zur Herstellung des Produktes zur Verfügung zu stellen, welches eine Rücknässung verhindert bei optimierter Nutzung einer Absorptionsleistung von superabsorbierenden Komponenten.

Diese Aufgabe wird mit einer absorbierenden Struktur nach Anspruch 1, einem Verfahren zur Herstellung einer absorbierenden Struktur nach Anspruch 17 und einer Verwendung einer Vorrichtung nach Anspruch 18 gelöst. Bevorzugte Ausführungsformen sind in den Unteransprüchen definiert. Ein oder mehrere Merkmale aus diesen Ausgestaltungen sind jedoch auch mit anderen Merkmalen aus der nachfolgenden Beschreibung zu weiteren Ausgestaltungen verknüpfbar und nicht allein auf die Jeweilige beanspruchte Weiterbildung beschränkt. Auch dienen die vorgeschlagenen jeweiligen Merkmale insbesondere auch der jeweiligen unabhängigen Ansprüche nur als erster Ansatz, wobei ein oder mehrere der Merkmale durch die nachfolgenden Merkmale ergänzt und/oder ausgetauscht werden können.

Es wird eine absorbierenden Struktur mit einer Folge an Schichten umfassend mindestens eine Flüssigkeitsaufnahmeschicht, eine nachfolgende Flüssigkeitsspeicherschicht mit superabsorbierendem Polymer SAP, vorzugsweise In Form von SAP-Partikeln und/oder SAP-Fasern, und eine nachfolgende Flüssigkeitsverteilungsschicht vorgeschlagen, wobei die Schichten verbunden sind und eine Lagenstruktur bilden. Dabei weisen zumindest die Flüssigkeitsspeicherschicht und die Flüssigkeitsverteilungsschicht zumindest jeweils ein Airlaid-Material als Hauptbestandteil auf, vorzugsweise eine Airlaid-Lage, welche Cellulosefasern umfasst. Zumindest weist die Fülssigkeitsspeicherschicht SAP-Partikel auf. Das superabsorbierende Polymer der Flüssigkeitsspeicherschicht, bevorzugt in der Form von SAP- Partikel und/oder SAP-Fasern, ragt partiell in die Flüssigkeitsverteilungsschicht hinein und tritt somit in direkten Kontakt zur Flüssigkeit, die In der Flüssigkeitsvertellungsschicht des Produktes verteilt wird. Das superabsorbierende Polymer bewirkt einen Rücksaugeffekt für die Flüssigkeit, die durch die Flüssigkeitsaufnahme- und die Flüssigkeitsspeicherschicht In die Flüssigkeltsvortellungsschicht eingetreten ist, wobei mehr als 20% der in der Flüssigkeitsspeicherschicht angeordneten SAP-Partikel und/oder SAP-Fasern in die Flüssigkeitsverteilungsschicht hineinragen.

Gemäß einer Weiterbildung ist vorgesehen, dass die Flüssigkeitsaufnahmeschicht ein Alrlaid-Material aufweist. Bevorzugt ist diese Lage zumindest zum überwiegenden Teil aus Cellulosefaser. Weiterhin kann vorgesehen sein, dass die Flüssigkeitsaufnahmeschicht thermoplastische Fasern aufweist, Diese Fasern können beispielsweise Klebefasern sein. Beispielsweise können Bico-Fasern, insbesondere Kern-Mantel-Fasern eingesetzt werden, bei denen der Mantel einen geringeren Schmelzpunkt aufweist als der Kern. Eine weitere Ausgestaltung sieht vor, dass die Flüssigkeitsaufnahmeschicht ein voluminöses Vlies aus thermoplastischen Fasern aufweist. Das voluminöse Vlies ist gemäß einer Ausgestaltung ein cardiertes Vlies. Eine Ausgestaltung sieht vor, dass es sich bei dem voluminösen Vlies um ein mit Heißluft verfestigtes Vlies aus thermoplastischen Fasern handelt. Gemäß einer Ausgestaltung werden Stapelfasern hierzu eingesetzt. Das voluminöse

Vlies kann Fasern aus Polyester, Polypropylen, Viscose und/oder Polyethylen aufweisen. Das voluminöse Vlies kann gemäß einer Ausgestaltung ein Flächengewicht von 30 bis 90 g/m² haben. Ein Beispiel eines verwendbares Material ist das unter der Bezeichnung Paratherm Loft 142/25 der Firma TWE angebotene Material.

Bevorzugt werden alle Lagen der absorbierenden Struktur in einem In-Line-Prozess hergestellt. Weiterhin besteht die Möglichkeit, dass zumindest eine Lage der absorbierenden Struktur zumindest vorgefertigt und dem Fertigungsprozess als Zwischenprodukt zugeführt wird. Dazu kann dieses Zwischenprodukt auf einem Wickler aufgerollt und nachfolgend an der Fertigungslinie wieder abgerollt und dem Verfahren zugeführt werden. Eine Ausführung sieht vor, dass zum Beispiel eine Lage zugeführt wird, die in der späteren absorbierenden Struktur als Flüssigkeitsaufnahmeschicht eingesetzt wird. Eine weitere Ausgestaltung sieht vor, dass zum Beispiel eine vorgefertigte Lage zugeführt wird, die in der später fertigen absorbierenden Struktur als Flüssigkeitsverteilungslage funktioniert. Eine Weiterbildung sieht vor, dass zumindest eine der Lagen auch mehrschichtig aufgebaut sein kann. Beispielsweise kann die Flüssigkeitsverteilungsschicht mit einer weiteren Schicht verbunden werden, vorzugsweise mit einer mit geringerer Porengröße als die Flüssigkeitsverteilungsschicht versehene Faserschicht, zum Beispiel einer Tissueschicht. Eine bevorzugte Ausgestaltung seiht vor, dass die Tissuelage direkt benachbart zu der Airlaid-Schicht angeordnet und mit dieser verbunden ist, die die Flüssigkeitsverteilungsschicht bildet. Das Tissue kann durch die kleinere Porengröße gegen über der Airlaid-Schicht eine Flüssigkeitsverteilung unterstützen. Die kleinere Porengröße bewirkt eine höhere Kapillarkraft. Das Tissue kann zum Beispiel eine Außenseite der absorbierenden Struktur bilden. Ein voluminöses Vlies aus thermoplastischen Fasern, bevorzugt Stapelfasern kann beispielsweise die andere Außenseite der absorbierenden Struktur bilden.

Die Verwendung eines thermoplastischen Materials in der Flüssigkeitsaufnahmeschicht ermöglicht beispielsweise eine Verbesserung der Wiederbenetzbarkeit. Thermoplastische Fasern verhindern die Aufnahme der Flüssigkeit in der Flüssigkeitsaufnahmeschicht. Statt dessen wird die Flüssigkeit an die Flüssigkeitsspeicherschicht weitergegeben. Dort wird die Flüssigkeit gespeichert, wobei ein Teil der Flüssigkeit auch in die Flüssigkeitsverteilungsschicht gelangen kann. Von dort wird die Flüssigkeit zum Beispiel entlang der Verteilungsschicht verteilt, bevor sie zurück in die Flüssigkeitsspeicherschicht gesaugt wird.

Sofern die Saugkraft eines superabsorbierenden Materials (SAP) deutlich höher ist als die Kapillarkraft der jeweiligen Schichten des Produktes, gelingt es so, Flüssigkeit, die durch den Gradienten der Kapillarkraft von der Flüssigkeitsaufnahmeschicht über die Flüssigkeitsspeicherschicht in die Flüssigkeitsverteilungsschicht gelangt und dort verteilt wurde, aus der Flüssigkeitsverteilungsschicht in die Flüssigkeitsspeicherschicht zu transportieren und dort in den superabsorbierenden Materialien zu speichern.

Eine Ausgestaltung sieht vor, dass die Flüssigkeitspeicherlage offene Kavitäten und/oder in ihrem Inneren großporige Bereiche aufweist. Diese können dazu genutzt werden, dass sich in diese hinein das superabsorbierende Material nach Aufnahme der Flüssigkeit hin ausdehnt, wenn es anschwillt. Die Freiräume dienen beispielsweise dazu, dass superabsorbierendes Material sich dorthinein verteilt, insbesondere während des Herstellungsprozesses der absorbierenden Struktur. Beispielsweise kann dieses durch Rüttelungen während des Herstellungsprozesses erfolgen.

Gemäß einer Ausgestaltung weist die absorbierende Struktur einen Gradienten hinsichtlich einer Porenstruktur auf, die ein Abfließen von der Flüssigkeitsaufnahmeschicht hin zur Flüssigkeitsverteilungsschicht unterstützt. Die Gradientenstruktur kann sich innerhalb einer Schicht wie aber auch über mehrere Schichten erstrecken. Der Gradient kann bevorzugt ein Ansteigen der Kapillarkraft bewirken. Ein Gradient ist zum Beispiel durch die Form der Ablage der Cellulosefasern einstellbar, durch zusätzliche Verdichtung und/oder durch Verringerung der Poren mittels zusätzlicher Mittel, zum Beispiel dem Zuführen von Flüssigkeit oder Binder, welches die Poren verkleinert oder aber teilweise verstopft. Beispielsweise ist dieses mittels einer Latexbenetzung möglich.

Eine Porengröße lässt sich zum Einen mittels bildgebender Verfahren ermitteln. So wird beispielsweise ein Schnitt entlang einer Längsfläche der Lagenstruktur ausgeführt. Dazu wird das Material beispielsweise so abgekühlt, dass es zerschneidbar ist, ohne dass aber beim Zerschneiden die Strukturen innerhalb der Schichten zerstört werden. Der Schnitt wird sodann fotografiert und mittels bildverarbeitender Verfahren ausgewertet. Ein Gradient entsteht zum Beispiel dadurch, dass quer zu einer Schicht die Porengröße abnimmt. Dazu wird die Anzahl der Poren im Schnitt gemessen und deren jeweilige Größe bestimmt. Durch Bildung des Verhältnisses beider Größen, nämlich aus Anzahl der Poren und der Summe von deren jeweiliger Größe, kann sodann eine mittlere Porengröße für diese Schicht ermittelt werden. Ein zu untersuchender Querschnitt sollte zumindest 20mm*20mm betragen.

Eine bevorzugte Weise, einen Schnitt herzustellen, ergibt sich unter Nutzung eines kryoskopischen Bruchs oder Schnitts mit extrem scharfer Klinge, bevorzugt mittels eines Mikrotoms. Eine so erstellte Schnittfläche kann anschließend ausgewertet werden, zum Beispiel durch eine Aufnahme der Schnittfläche und anschließender bevorzugt automatischer Auszählung. Auch besteht die Möglichkeit, eine Rasterelektronenmikroskopaufnahme anzufertigen, die anschließend ausgewertet wird. Letzteres wird beispielsweise durch das Sächsische Institut für Textilforschung, das STFI in Chemnitz in standardisierter Weise ausgeführt.

Neben der Bildung eines Gradienten entlang einer Schicht kann ein Gradient auch quer dazu in einer Schicht vorhanden sein. Dieser kann zum Beispiel ebenfalls durch ein Verfahren wie oben bestimmt werden.

Ein erster Gradient im Vergleich der einzelnen Schichten kann beispielsweise mittels einer Beurteilung eines jeweiligen Querschnitts senkrecht zur Längserstreckung einer Schicht beurteilt werden. Dieser Gradient wird daher als Gesamtschichtgradient bezeichnet. Ein zweiter Gradient im Vergleich der einzelnen Schichten kann beispielsweise dadurch ermittelt werden, dass die oberste und die unterste Querschicht jeweils einer Schicht genommen wird. Diese werden beispielsweise miteinander verglichen. Daraus lässt sich eine Information entnehmen, ob eine Schicht einen höheren Widerstand gegenüber einem Austritt von Flüssigkeit nach oben oder nach unten aufweist. Auch können beide Schichten jeweils hinsichtlich ihrer jeweiligen mittleren Porengröße bestimmt werden. Damit ist der jeweilige Grenzwert einer mittleren Porengröße der Schicht bestimmt. Zum Beispiel lässt sich aus den beiden Grenzwerten wiederum ein Mittelwert bilden, der als Grenzwertgradient bezeichnet wird. Bevorzugt ist es, wenn der Grenzwertgradient und der jeweilige Grenzwert über eine Lagenstruktur betrachtet jeweils zunehmen.

Eine weitere Ausgestaltung sieht vor, dass beispielsweise die mittlere Porengröße in einem Übergang von einer Schicht zur nächsten Schicht von der Flüssigkeitsaufnahmeschicht bis zur Flüssigkeitsverteilungsschicht hin jeweils abnimmt.

Die absorbierende Struktur weist den Vorteil auf, dass Flüssigkeit, die während des Gebrauchs der absorbierenden Struktur von der Flüssigkeitsaufnahmeschicht in die Flüssigkeitsspeicherschicht läuft, aufgenommen und vorzugsweise auch verteilt wird, wodurch Rücknässungserscheinungen durch ein Auslaufen von Flüssigkeit vermieden werden. Eine Verteilung von Flüssigkeit erfolgt aber nicht nur in eine Längsrichtung innerhalb der Flüssigkeitsverteilungsschicht. Vielmehr hat sich herausgestellt, dass mittels einer gezielten Einstellung, Anordnung und Verteilung der SAP-Partikel und/oder SAP-Fasern zwischen den beiden Schichten eine Rückströmung einstellen lässt, bei der die Flüssigkeit mittels der SAP-Partikel und/oder SAP-Fasern in der Flüssigkeitsverteilungsschicht schon aufgenommen und sodann durch die SAP-Partikel und/oder SAP-Fasern in die Flüssigkeitsspeicherschicht zurückgelangt. Dieser Mechanismus kann beispielsweise durch eine Einstellung einer Porosität des verwendeten Airlaid-Materials unterstützt werden. Gemäß einer Ausgestaltung kann hierbei ein Material eingesetzt werden, wie es zum Beispiel aus der WO 00/74620 hervorgeht. Ein derartiger Gradient bezüglich Dichte und/oder Porosität kann beispielsweise auch hier eingesetzt werden. Bei der in WO 00/74620 beschriebenen Struktur aus Flüssigkeitsaufnahmeschicht, Flüssigkeitsspeicherschicht und Flüssigkeitsverteilungsschicht steht neben der Gravitationskraft nur die Kapillarkraft, die von der Flüssigkeitsaufnahmeschicht zur Flüssigkeitsverteilungsschicht gerichtet ist, zur Verfügung. Somit ist in den in WO 00/74620 beschriebenen Strukturen eine Rückströmung der Flüssigkeit von der Flüssigkeitsverteilungsschicht in die Flüssigkeitsspeicherschicht physikalisch nicht möglich. Die nunmehr vorgeschlagene technische Lehre führt aber zur Ermöglichung einer Ausprägung einer Rückströmung.

Eine Ausgestaltung sieht vor, dass die SAP-Partikel und/oder SAP-Fasern unregelmäßig in die Flüssigkeitsverteilungsschicht aus der Flüssigkeitsspeicherschicht hineinragen. Eine weitere Ausgestaltung sieht vor, dass es Bereiche gibt, innerhalb der kein SAP-Partikel und/oder SAP-Faser in die Flüssigkeitsverteilungsschicht aus der Flüssigkeitsspeicherschicht hineinragt, während in benachbarten Bereichen zumindest die überwiegende Anzahl der SAP-Partikel und/oder SAP-Fasern, d.h. mindestens 50% in die Flüssigkeitsverteilungsschicht aus der Flüssigkeitsspeicherschicht hineinragt. Eine weitere Ausgestaltung sieht vor, dass es Bereiche gibt, innerhalb der weniger SAP-Partikel und/oder SAP-Fasern in die Flüssigkeitsverteilungsschicht aus der Flüssigkeitsspeicherschicht hineinragen gegenüber benachbarter Bereiche, insbesondere gegenüber Bereiche, bei der zumindest die überwiegende Anzahl der SAP-Partikel und/oder Fasern in die Flüssigkeitsverteilungsschicht aus der Flüssigkeitsspeicherschicht hineinragen.

Bevorzugt ist es, wenn die absorbierende Struktur SAP-Partikel und/oder SAP-Fasern aufweist, die zumindest zu einem Drittel einer Längserstreckung in die Flüssigkeitsspeicherschicht hineinragen. Ein Hineinragen der Längserstreckung kann zum Beispiel davon abhängig gemacht werden, welche Dicken die verschiedenen Schichten aufweisen. Bevorzugt ist es, wenn die SAP-Partikel und/oder SAP-Fasern zumindest soweit in die Flüssigkeitsverteilungslage hineinragen, dass sie über ein Zehntel, bevorzugt über ein Viertel einer Dicke der Flüssigkeitsverteilungslage tief in diese hineinragen.

Bei einer SAP-Faser wird die Länge dadurch bestimmt, dass deren Erstreckung von Anfang zum Ende als kürzeste Strecke in der Struktur gemessen wird. Dieses gilt auch für gewundene SAP-Fasern. SAP-Fasern können annähernd gerade wie auch in einer gewundenen Struktur angewandt werden.

Eine Ausgestaltung sieht vor, dass bei einer absorbierenden Struktur mehr als 40% der in der Flüssigkeitsspeicherschicht angeordneten SAP-Partikel und/oder SAP-Fasern in die Flüssigkeitsverteilungsschicht hineinragen. Eine weitere Ausgestaltung sieht vor, dass mehr als 40% aber weniger als 70% der in der Flüssigkeitsspeicherschicht angeordneten SAP-Partikel und/oder SAP-Fasern In die Flüssigkeitsverteilungsschicht hineinragen.

Bevorzugt ist des Weiteren wenn bei der absorbierenden Struktur die in die Flüssigkeitsverteilungsschicht hineinragenden SAP-Partikel und/oder SAP-Fasern nach Flüssigkeitsaufnahme sich mehr in der Flüssigkeitsspeicherschicht denn in der Flüssigkeitsverteilungsschicht vergrößert haben. So kann beispielsweise die Schwellfähigkeit eines SAP-Partikels oder einer SAP-Faser derart sein, dass es sich ausdehnt in Richtung des geringeren Widerstands. Weist die Flüssigkeitsspeicherlage einen geringeren Widerstand auf, zum Beispiel über eine geringere Dichte, eine offenporigere Struktur, eine geringere Bindungshaftung der Fasern untereinander und/oder durch andere Mittel, die einen Widerstand gegen Verdrängung bei einem Bereich der Flüssigkeitsspeicherschicht und bevorzugt den Fasern hervorrufen können, kann ein anschwellender SAP-Partikel oder eine SAP-Faser beispielsweise nicht nur bevorzugt in der Flüssigkeitsspeicherschicht anschwellen. Beispielsweise kann dadurch auch eine Bewegung des anschwellenden SAP-Partikels oder der SAP-Faser zurück in die Flüssigkeitsspeicherschicht erfolgen. Gemäß einer Weiterbildung ist vorgesehen, dass In die Flüsslgkeilsverteilungsschicht hineinragende SAP-Partikel und/oder SAP-Faser nach Flüssigkeitsaufnahme sich in die Flüssigkeitsverteilungsschicht zumindest teilweise zurückgezogen haben.

Das superabsorbierende Material wie zum Beispiel in Form von oben schon dargestellten SAP-Partikeln und/oder SAP-Fasern ist schwellfähig und geht in der Regel in einen gelartigen Zustand über. Sie können dadurch nicht nur Wasser speichern. Vielmehr sind die SAP-Partikel bei einer wie oben beschriebenen Anordnung in der Lagenstruktur in der Lage, einen Saugfluss zu erzeugen und damit beispielsweise als Drainagematerial für die Flüssigkeitsverteilungsschicht zu dienen.

Chemisch gesehen kann es sich bei SAP um Copolymere handeln, die zum Beispiel Acrylsäure und Natriumacrylat aufweisen, wobei das Verhältnis der beiden Monomeren zueinander variieren kann. Zusätzlich werden beispielsweise Quervernetzer bei der Polymerisierung zugesetzt, die das gebildete langkettige Polymer stellenweise untereinander durch chemische Brücken verbinden. Je nach Vernetzungsgrad können die die Eigenschaften des Polymers eingestellt werden. Eine Ausgestaltung sieht zum Beispiel vor, dass zwei verschiedene SAP-Materialien, zum Beispiel zwei verschiedene SAP-Partikel, zwei verschiedene SAP-Fasern und/oder voneinander verschiedene SAP-Partikel und SAP-Fasern mit voneinander verschiedenen Eigenschaften zum Einsatz gelangen. So kann die Verschiedenheit zum Beispiel in der Flüssigkeitsaufnahmefähigkeit, der Schnelligkeit der Flüssigkeitsaufnahme, der Schwellung selbst bei Flüssigkeitsaufnahme, einer Zeitverzögerung bis zum Flüssigkeitsaufnahmebeginn, einer Flüssigkeitsaufnahmerate oder einem sonstigen Parameter bestimmt sein. Verschiedene SAP-Materialien können gemischt wie auch getrennt voneinander in verschiedenen Bereichen angeordnet sein. Die getrennten Bereiche können nicht nur in MD- und CD-Richtung einer Verarbeitung auf eine Airlaid-Herstellungsvorrichtung bezogen angeordnet sein. Vielmehr kann auch eine Anordnung entlang einer Dicke des Materials sich unterscheiden, insbesondere können dadurch unterschiedliche Bereiche ausgeformt werden.

Zum Beispiel können SAP-Materialien zum Einsatz gelangen, wie sie beispielsweise aus der EP 0810 886 hervorgehen, insbesondere auch aus dem dort zitierten Stand der Technik, auf die im Rahmen der Offenbarung vollständig verwiesen wird. Eine Ausgestaltung sieht beispielsweise vor, dass SAP-Partikel eine Beschichtung aufweisen. Die Beschichtung kann sich beispielsweise in Verbindung mit einer Flüssigkeit erst auflösen, um damit eine Aufnahme der Flüssigkeit durch das SAP-Partikel überhaupt zu ermöglichen. Dieses ist eine Möglichkeit, wie beispielsweise eine Zeitverzögerung eines Aufsaugens und bevorzugt Zurücksaugens von Flüssigkeit in der Flüssigkeitsverteilungslage in die Flüssigkeitsspeicherlage einstellen lässt. Beispielsweise kann in der Flüssigkeitsspeicherschicht ein erstes SAP-Material angeordnet sein, dass unbeschichtet ist und ein zweites SAP-Material, das beschichtet ist. Durch den Flüssigkeitskontakt bindet zuerst das erste SAP-Material diese. Überschießende Flüssigkeit tritt in die Flüssigkeitsverteilungsschicht ein, wobei das zweite SAP-Material erst mit Zeitverzögerung nach Vorbeifließen bzw. Kontaktierung mit der Flüssigkeit aktiviert wird. Das zweite SAP-Material ist damit in der Lage, insbesondere als Drainage wirkend, Flüssigkeit zurücksaugen zu können und hierbei einen Rückstromfluss in die Flüssigkeitsspeicherschicht aufbauen zu können. Beispielsweise kann vorgesehen sein, dass das zweite SAP-Material im Wesentlichen in die Flüssigkeitsverteilungsschicht hineinragt, das erste Material hingegen kaum oder gar nicht. Dieses wird beispielsweise durch den Lagenaufbau eingestellt wie auch durch denn anschließenden Druck auf die erzeugte Lagenstruktur.

Weiterhin kann SAP-Material eingesetzt werden, wie es aus der DE 10 2004 015 686 A1, der DE 698 217 94 und/oder der DE 10 2004 005 417 A1 jeweils hervorgeht, insbesondere in Bezug auf den Aufbau und die Struktur, die Geometrie des superabsorbierenden Polymers wie auch der dabei verwendeten Materialien und Herstellungsweisen. Auf diese Druckschriften wird im Rahmen der Offenbarung beispielhaft verwiesen. Eine weitere Ausgestaltung sieht vor, dass die SAP-Partikel granulatförmig aber auch eine andere Geometrie aufweisen können, zum Beispiel faserförmig, rund oder andersförmig sein können. Fasern mit einem Gehalt an Superabsorber gehen zum Beispiel aus der DE 102 32 078 A1 wie auch aus der DE 102 51 137 A1 hervor. Auch auf diese wird im Rahmen der Offenbarung verwiesen.

Eine Weiterbildung sieht vor, dass die Eigenschaft des SAP gezielt eingestellt wird, bevorzugt gezielt auf die aufzunehmende Flüssigkeit, aber beispielsweise auch auf das absorbierende Produkt und die dort jeweils vorherrschenden Bedingungen für das SAP. Es gibt den Ansatz AAP, ausgeschrieben Absorption against pressure, um zu zeigen, wie SAP trotz Druck absorbieren kann. Hierzu existiert die WSP-EDANA-Methode WSP 242.2 (05). Des Weiteren kann das SAP mittels der sogenannten Free Swell Capacity gemäß der Methode WSP 240.2 (05) beschrieben werden. Auch besteht die Möglichkeit, die Zentrifugenretentionskapazität (CRC) WSP 241.2 (05) zu betrachten. Eine bevorzugte Ausgestaltung sieht vor, eine Charakterisierung des SAPs anhand des Verhältnisses von " Absorption against pressure" zu "Free Swell Capacity" zu beschreiben. Gemäß einer Ausgestaltung wird ein SAP mit einem CRC-Wert von 30 bis 35g/g und einem AAP -Wert je nach Vernetzung zwischen 18 bis 24g/g.

Bevorzugt wird der Quelldruck des SAP zum Ansaugen von Flüssigkeit bevorzugt aus der Flüssigkeitsverteilungsschicht in die Flüssigkeitsspeicherschicht ausgenutzt. So weist das SAP beispielsweise einen Quelldruck in Höhe von 6 bis 8 bar auf, entsprechend damit ca. 80 bis 90 m Wassersäule. Dieser Wert ist sehr viel höher gegenüber einer Kapillarkraft, die sich beispielsweise für SAP bei kleiner 1 m Wassersäule einstellen kann. Der Quelldruck kann daher als eine Vertikalkraft genutzt werden, um Flüssigkeit von der Flüssigkeitsverteilungsschicht in die Flüssigkeitsspeicherschicht zurückzuholen.

In einer Ausführungsform ist die absorbierende Struktur als Lagenstruktur ausgebildet, wobei die Lagenstruktur zumindest einen Grenzbereich aufweist und wobei die Lagenstruktur innerhalb der Flüssigkeitsverteilungsschicht und/oder der Flüssigkeitsspeicherschicht und/oder der Flüssigkeitsaufnahmeschicht und/oder in einem Grenzbereich zwischen der Flüssigkeitsverteilungsschicht und/oder der Flüssigkeitsspeicherschicht und/oder der Flüssigkeitsaufnahmeschicht Kavitäten aufweist.

Diese Kavitäten können zum Beispiel bei der Herstellung der absorbierenden Struktur entstehen. Zum Beispiel, indem die Lagen verbunden werden, wodurch die Lagenstruktur gebildet wird und während des Bindeprozesses eine zumindest in Maschinenrichtung orientierte Versteckung der Lagenstruktur erfolgt. Die Airlaid-Lagen werden durch Thermobondieren, Verwendung von Bindern oder durch Kalandrieren verbunden und verdichtet. Nach diesem Bindeprozess kann sich die so erhaltene Lagenstruktur wieder entspannen, wodurch sich die Kavitäten innerhalb der Airlaid-Lagen oder an deren Grenzflächen ebenfalls entspannen und in der absorbierenden Struktur als vorzugsweise unregelmäßige Hohlräume erkennbar sind, in denen vorzugsweise keine Cellulosefasern und vorzugsweise auch keine SAP-Partikel und/oder SAP-Partikel angeordnet sind.

Bevorzugt sind diese Kavitäten in der Flüssigkeitsspeicherschicht größer ausgebildet, als in der Flüssigkeitsaufnahmeschicht und/oder in der Flüssigkeitsverteilungsschicht.

Eine weitere Ausführungsform sieht vor, dass die absorbierende Struktur derart ausgebildet ist, dass zumindest ein Teil der Cellulosefasern der Airlaid-Lagen der Flüssigkeitsverteilungsschicht und/oder der Flüssigkeitsspeicherschicht und/oder der Flüssigkeitsaufnahmeschicht in dem Grenzbereich miteinander vermischt sind.

Vorzugsweise gehen die Airlaid-Lagen der Flüssigkeitsverteilungsschicht und/oder der Flüssigkeitsspeicherschicht und/oder der Flüssigkeitsaufnahmeschicht innerhalb des Grenzbereiches ineinander über.

Eine Weiterbildung sieht vor, dass die Airlaid-Lagen der Flüssigkeitsverteilungsschicht und/oder der Flüssigkeitsspeicherschicht und/oder der Flüssigkeitsaufnahmeschicht innerhalb der Lagenstruktur nicht voneinander unterscheidbar sind. Bevorzugt werden gleiche oder gleichartige Cellulosefasern eingesetzt. Beispielsweise werden die Fasern an getrennten Stationen abgelegt. Nach der Ablage sind aber zum Beispiel zwei Lagen nicht mehr so anhand einer scharfen Phasengrenze voneinander trennbar. Vielmehr gehen diese ineinander über bzw. sind nicht mehr als zwei verschiedene Lagen in einer Querschnittsansicht identifizierbar.

Die absorbierende Struktur kann derart ausgebildet sein, dass die Flüssigkeitsverteilungsschicht eine erste und eine zweite Oberfläche aufweist, wobei die erste Oberfläche in Kontakt mit der Flüssigkeitsspeicherschicht steht und wobei die Flüssigkeitsverteilungsschicht an ihrer zweiten Oberfläche stärker verdichtet ist, als an ihrer ersten Oberfläche.

Es hat sich als vorteilhaft erwiesen, dass die Flüssigkeitsspeicherschicht eine höhere Dichte als die Flüssigkeitsverteilungsschicht und/oder die Flüssigkeitsaufnahmeschicht aufweist. Eine Dichteberechnung erfolgt hier als Gesamtdichte der Schicht, d.h. alle Bestandteile einer Schicht gehen ein. Dazu wird die Schicht gemessen und bezogen auf die Dimension der Schicht die Dichte ausgerechnet. Im Ergebnis ist damit eine gemittelte Dichte bestimmbar, die die Schicht charakterisiert.

Eine Weiterbildung sieht vor, dass die SAP-Partikel und/oder SAP-Fasern aus der Flüssigkeitsspeicherschicht in die Flüssigkeitsverteilungsschicht hineinragen. Es hat sich herausgestellt, dass über die in der Lagenstruktur und innerhalb der einzelnen Airlaid-Lagen wirkende Kapillarkraft zunächst Flüssigkeit von der Flüssigkeitsaufnahmeschicht zur Flüssigkeitsverteilungsschicht hinfließt. Durch die aus der Flüssigkeitsspeicherschicht in die Flüssigkeitsverteilungsschicht hineinragenden SAP-Partikel und/oder SAP-Fasern wird ein Saugeffekt erzeugt, wodurch Flüssigkeit aus der Flüssigkeitsverteilungslage zurück in die Flüssigkeitsspeicherlage fließt. Gemäß einer Ausgestaltung ist vorgesehen, dass bei einer erstmaligen Flüssigkeitsaufgabe auf die Flüssigkeitsaufnahmeschicht die Saugwirkung, die durch die SAP-Partikel und/oder SAP-Fasern hervorgerufen wird, zeitlich verzögert zur Saugwirkung durch Kapillareffekte stattfindet. Gemäß einer weiteren Ausgestaltung ist vorgesehen, dass bei mehrmaliger Flüssigkeitsaufgabe es zur Überlagerung von Transportvorgängen für die Flüssigkeit kommen kann, wobei bevorzugt der Transport durch Kapillarkräfte in der Regel schneller erfolgt als der zeitlich später einsetzende Rücktransport von Flüssigkeit aus der Flüssigkeitsverteilungslage in die Flüssigkeitsspeicherlage durch die superabsorbierenden Materalien.

Es hat sich ebenfalls als vorteilhaft erwiesen, eine absorbierende Struktur derart zu gestalten, dass zumindest eine Flüssigkeitsaufnahmeschicht vorgesehen ist und eine Flüssigkeitsspeicherschicht als mehrlagige Schicht, vorzugsweise zweilagige Schicht ausgebildet ist. Beispielsweise kann die Flüssigkeitsspeicherschicht gebildet sein aus zumindest
- einer Lage, umfassend eine Airlaid-Lage, vorzugsweise beinhaltend Cellulosefasern und SAP-Partikel und/oder SAP-Fasern und zumindest einer weiteren Lage, , beinhaltend SAP-Partikel oder
- zwei Lagen, umfassend jeweils eine Airlaid-Lage, vorzugsweise beinhaltend Cellulosefasern und SAP-Partikel und/oder SAP-Fasern.

Das Einbringen von superabsorbierenden Polymeren, bevorzugt in der Form von SAP-Partikeln und/oder SAP-Fasern, in einer weiteren Lage der absorbierenden Struktur ermöglicht beispielsweise, dass diese sowohl die Funktion einer Flüssigkeitsspeicherschicht als auch einer Flüssigkeitsverteilungsschicht übernehmen kann. Die Lage weist gemäß einer Ausgestaltung vorzugsweise die höchste Dichte innerhalb der Flüssigkeitsspeicher- bzw. Flüssigkeitsverteilungsschicht der Lagen-Struktur auf und somit ein sehr gutes Flüssigkeitsverteilungsverhalten. Zudem kann die Lage zur Verbesserung eines Dehnungsverhaltens der Lagenstruktur beitragen, zum Beispiel auch hinsichtlich einer elastischen Eigenschaft. Ein weiterer Vorteil ergibt sich daraus, dass sich die SAP-Partikel und/oder SAP-Fasern innerhalb der Lage gut ausdehnen können und genügend Raum zum Quellen zur Verfügung steht, wodurch das Absorptionsvermögen der Lagenstruktur verbessert wird. Zudem kann mit einer zusätzlichen Lage ein höherer Anteil an SAP-Partikeln und/oder SAP-Fasern eingebettet werden. Damit kann ein derartiges Produkt insbesondere den Anforderungen an Inkontinenzprodukte gerecht werden.

Die jeweiligen Lagen können dabei gleiche oder unterschiedliche Arten an Cellulosefasern und/ oder SAP-Partikel und/oder SAP-Fasern aufweisen. Auf diese Weise kann das Aufnahmeverhalten der Einzellagen der Lagenstruktur für Flüssigkeiten definiert eingestellt werden.

Beispielsweise können in einer Lage hochpermeable SAP-Partikel und/oder SAP-Fasern eingesetzt werden, die zusammen mit SAP-Partikeln und/oder SAP-Fasern in einer weiteren Lage einen zweistufigen Absorptions- und Speichereffekt bewirken. Beispielsweise könnten in einer Lage, die der Flüssigkeitsaufnahmeschicht zugewandt ist, SAP-Partikel und/oder SAP-Fasern mit hoher Absorptionsfähigkeit und in einer weiteren Lage semipermeable SAP-Partikel und/oder SAP-Fasern vorgesehen sein. Hierdurch kann eine Pufferfunktion in der weiteren Lage erzeugt werden, die sich speziell bei mehrmaligem Aufgeben einer Flüssigkeit als vorteilhaft erweist.

In einer Weiterbildung weist die Flüssigkeitsaufnahmeschicht zumindest Cellulosefasern und Bikomponentenfasern auf, wobei die Flüssigkeitsspeicherschicht zumindest Cellulosefasern und SAP-Partikel und/oder SAP-Fasern umfasst.

Eine Ausführungsform sieht vor, dass die Bikomponentenfasern eine Kern-Mantelstruktur aufweisen. Es ist ebenfalls vorgesehen, dass die Bikomponentenfasern zumindest ein PET umfassen. Vorteilhafterweise umfassen die Bikomponentenfasern zumindest ein Polyethylen, vorzugsweise ein LDPE oder ein LLDPE. In einer Bikomponentenfaser mit einer Kern-Mantel-Struktur ist ein PET oder ein Polypropylen enthaltendes Polymer im Kern und ein Polyethylen enthaltendes Polymer im Mantel vorgesehen. Die Bikomponentenfasern werden bevorzugt als Bindefasern genutzt. Durch Erwärmung werden diese zumindest soweit aufgeweicht, dass sie eine klebrige Oberfläche bilden, an der Cellulosefasern wie auch andere Komponenten der Schicht aber auch Komponenten benachbarter Schichten festgefügt werden bei Abkühlung. Gemäß einer Ausgestaltung können cellulosebindende Fasern eingesetzt werden, wie sie aus der DE69808061 hervorgehen, auf die im Rahmen der Offenbarung verwiesen wird.

Eine weitere Ausführungsform sieht vor, dass in der Flüssigkeitsaufnahmeschicht zumindest Cellulosefasern und Bikomponentenfasern vorgesehen sind, die Flüssigkeitsspeicherschicht zumindest Cellulosefasern und SAP-Partikel und/oder SAP-Fasern aufweist, die Flüssigkeitsverteilungsschicht zum überwiegenden Teil Cellulosefasern aufweist und eine Bindemittelschicht vorgesehen ist, vorzugsweise eine Latexschicht.

Die einzelnen Lagen der absorbierenden Struktur können
- jeweils die gleiche Sorte an Cellulosefasern,
- jeweils unterschiedliche Sorten an Cellulosefasern,
- Mischungen hieraus,
- chemisch und/oder physikalisch behandelte Cellulosefasern,
- unbehandelte Cellulosefasern,
- Mischungen aus behandelten und unbehandelten Cellulosefasern,
- synthetische Fasern allein oder in Mischung mit Cellulosefasern in behandelter oder unbehandelter Form, sowie
- Fasern mineralischen Ursprungs allein oder in Mischung mit synthetischen und/oder Cellulosefasern
aufweisen. Einzelne Lagen können auch ausschließlich Cellulosefasern aufweisen.

Eine einzelne Lage kann hierbei als Flüssigkeitsverteilungsschicht oder Flüssigkeitsspeicherschicht oder Flüssigkeitsaufnahmeschicht innerhalb der Lagenstruktur der absorbierenden Struktur ausgebildet sein. Eine einzelne Lage kann auch als eine Lage in einer mehrlagigen Flüssigkeitsverteilungsschicht oder Flüssigkeitsspeicherschicht oder Flüssigkeitsaufnahmeschicht ausgebildet sein.

Der Begriff "Cellulosefasern" wird im Rahmen der Offenbarung nicht einschränkend verstanden. Einsetzbar sind jegliche Naturfasern, die in der Lage sind oder durch eine chemisch und/oder physikalische Behandlung in die Lage versetzt worden sind, Flüssigkeiten aufzunehmen und vorzugsweise auch zu binden. Durch eine ebensolche Behandlung können auch synthetische Fasern und Fasern mineralischen Ursprungs aufbereitet sein.

Unter einer chemischen Behandlung werden beispielsweise
- Waschvorgänge, Extrahiervorgänge,
- Bleichvorgänge,
- Färbevorgänge,
- Fibrillierungsvorgänge unter Verwendung von Lösemitteln,
- Oberflächenbehandlung, vorzugsweise zur Hydrophilierung, Erhöhung der Festigkeit oder Elastizität, beispielsweise durch Sprühen, Tauchen, Tränken, Waschen
und Ähnliches verstanden.

Eine physikalische Behandlung kann durch
- Zerkleinerung und Fibrillierung, beispielsweise Schneiden, Mahlen, Zerfasern,
- Klassierung, beispielsweise Windsichten
erfolgen.

Es hat sich als zweckmäßig erwiesen, dass die Flüssigkeitsaufnahmeschicht eine Airlaid-Lage umfasst, die im Wesentlichen chemisch und/oder physikalisch unbehandelte Cellulosefasern aufweist. Gemäß einer weiteren Ausgestaltung sind die Cellulosefasern der Flüssigkeitsspeicherschicht chemisch und/oder physikalisch unbehandelt.

Eine weitere Ausführungsform sieht vor, dass die Flüssigkeitsaufnahmeschicht eine cellulosefaserfreie oder eine mit einem nur geringen Cellulosefaseranteil versehene Aufnahmeschicht als Airlaid-Lage geformt ist, die z.B. thermoplastische Fasern, zum Beispiel Stapelfasern aufweist, und die Flüssigkeitsverteilungsschicht eine Airlaid-Lage umfasst, die im Wesentlichen chemisch und/oder physikalisch behandelte Cellulosefasern aufweisen. Durch Verwendung thermoplastischer Fasern als überwiegender Faserbestandteil der Aufnahmeschicht kann ein erneutes Benetzen mit Flüssigkeit und Weiterleiten in die Flüssigkeitsspeicherlage verbessert werden. Hierzu können angepasst an einen Einsatzzweck die Fasern der Flüssigkeitsaufnahmeschicht entsprechend ausgerüstet sein, zum Beispiel hydrophob.

Besonders bevorzugte Cellulosefasern, Southern-Pine-Pulptypen der Fa. Koch Cellulose LLC sind beispielsweise in einem Pulping-Prozesses und einem Bleich-Prozess hergestellt und nachbehandelt worden. Die Fasern weisen beispielsweise eine durchschnittliche Faserlänge von 2,7 mm, eine Dichte von 0,9 g/cm³, eine Bruchfestigkeit von 414 kPa, eine Feuchtigkeit von 8%, eine zerfaserbarer Anteil von über 99,5 %, ein spezifisches Absorptionsvermögen von 1,5 s/g auf.

Diese Fasern eignen sich auf Grund ihrer Behandlung speziell für den Einsatz in den äußeren Lagen der absorbierenden Struktur, das heißt, in der Flüssigkeitssaufnahme- und der Flüssigkeitsverteilungsschicht. Je nach Art des Aufschluss-Prozesses für die Fasern und des Bleich-Prozesses sind definierte Eigenschaftskombinationen in den Cellulosefasern erreichbar.

Es ist vorgesehen, dass die chemisch und/oder physikalische Behandlung der Cellulosefasern der Airlaid-Lage der Flüssigkeitsaufnahmeschicht von der chemisch und/oder physikalische Behandlung der Cellulosefasern der Airlaid-Lage der Flüssigkeitsverteilungsschicht verschieden ist.

In der Flüssigkeitsspeicherlage werden vorzugsweise unbehandelte Fasern, vorzugsweise der Southern Pine eingesetzt. Dies hat unterschiedliche Gründe. Der Pulp verliert durch das Zusetzen von Behandlungsmitteln, insbesondere Oberflächenbehandlungsmitteln an Absorptionsfähigkeit. Um in einer Flüssigkeitsspeicherlage eine bestmögliche Absorption zu gewährleisten, wird bevorzugt ein unbehandelter Pulptyp eingesetzt. Dieser lässt sich auch am besten im Prozess verdichten, da die unbehandelten Fasern gut zusammenhaften.

In den äußeren Schichten der absorbierenden Struktur, die vorzugsweise durch eine Flüssigkeitsaufnahmeschicht und eine Flüssigkeitsverteilungsschicht gebildet werden, wird prozessbedingt ein behandelter Cellulose-Pulp-Typ eingesetzt, um Anhaftungen an den in einem Bindeprozess verwendeten Vorrichtungen, beispielsweise einer Prägewalze in einer Kalanderanordnung zu vermeiden. Die Oberflächenbehandlung der Fasern reduziert zudem die Haftung zwischen den Fasern und fördert somit deren Verdichtbarkeit.

Cellulosefasern der Fa. Koch Cellulose LLC des Typs GP4821 eignen sich beispielsweise für den Einsatz in einer äußeren Lage einer absorbierenden Struktur, da deren voluminöse Struktur beispielsweise an die Anforderungen eines Kalandrierprozesses zur Verdichtung einer Lagenstruktur angepasst ist

Gemäß einer Ausführungsform wird eine absorbierende Struktur vorgeschlagen, worin
- die Flüssigkeitsaufnahmeschicht zumindest Cellulosefasern in einem Bereich zwischen 60 Gew.-% bis 70 Gew.-% und Bikomponentenfasern in einem Bereich zwischen 30 Gew.-% bis 40 Gew.-%, bezogen auf das Gesamtgewicht der Flüssigkeitsaufnahmeschicht aufweist,
- die Flüssigkeitsspeicherschicht zumindest Cellulosefasern und SAP-Partikel und/oder SAP-Fasern im Bereich zwischen 15 Gew.-% bis 35 Gew.-%, bezogen auf das Gesamtgewicht der absorbierenden Struktur aufweist und
- die Flüssigkeitsverteilungsschicht zum überwiegenden Teil Cellulosefasern, vorzugsweise 100 Gew.-%, bezogen auf ihr Gesamtgewicht aufweist.

Zudem kann die absorbierende Schicht eine weitere Binderschicht, beispielsweise eine Latexschicht, aufweisen, die auf der Flüssigkeitsverteilungsschicht angeordnet ist.

Ein mögliches Verfahren zur Herstellung einer absorbierenden Struktur kann zum Beispiel zumindest die folgenden Schritte aufweisen, wobei die Reihenfolge der Schritte änderbar ist:
Ablegen einer ersten Lage, bevorzugt einer Airlaid-Lage, die vorzugsweise zumindest Cellulosefasern und Bikomponentenfasern umfasst, und/oder einer hochvoluminösen Vlieslage aus thermoplastischen Fasern, zur Ausbildung einer Flüssigkeitsaufnahmeschicht,
Ablegen einer zweiten Airlaid-Lage zur Ausbildung einer Flüssigkeitsspeicherschicht, die zumindest Cellulosefasern und SAP-Partikel und/oder SAP-Fasern umfasst,
Ablegen einer dritten Airlaid-Lage zur Ausbildung einer Flüssigkeitsvertellungsschicht,
vorzugsweise Aufbringen einer Bindemittelschicht, vorzugsweise einer Latexschicht auf eine Lage, vorzugsweise auf die so erhaltene Lagenstruktur,
vorzugsweise Durchführen der Lagenstruktur durch ein Heizmittel, um die Lagenstruktur zu binden,
Zuführen zumindest einer Lage, bevorzugt der Lagenstruktur zu einem Kalander, umfassend mindestens eine Glattwalze und eine Gegenwalze vorzugsweise mit Erhebungen, die einen Kalanderspalt bilden,
Verdichtung der zumindest einen Lage, bevorzugt der Lagenstruktur im Kalanderspalt, wobei mehr als 20% der In der Flüssigkeitsspeicherschicht angeordneten SAP-Partikel und/oder SAP-Fasern aus der Flüssigkeitsspeicherschicht in die Flüssigkeitsverteilungsschicht hineinragen und einen flüssigkeitsabführenden Kontakt zwischen Flüssigkeitsspeicherschicht und Flüssigkeitsverteilungsschicht bilden.

Insbesondere wird mit einer derartigen Vorrichtung eine absorbierende Struktur hergestellt, wie sie oben und noch nachfolgend weiter beschrieben wird.

Eine Ausgestaltung sieht beispielsweise vor, dass zuerst eine Flüssigkeitsverteilungsschicht, zum Beispiel eine pulpbaslerte Vorteilungsschicht, von einem ersten Formierkopf auf ein Siebband abgelegt bzw.abgezogen wird. Beispielsweise kann hierzu ergänzend vorab und/oder später eine Tissueschicht zugeordnet werden. Auf der Flüssigkeitsverteilungsschicht aufbauend wird eine Speicherschicht und schließlich eine Aufnahmeschicht angeordnet. Aufgrund dieser Konstellation ist beispielsweise ein Kalander vorgesehen, der eine Walze mit den Prägeerhebungen unten platziert aufweist, so dass die Erhebungen in direkten Kontakt mit der Verteilungsschicht bzw. dem Tissue geraten. Ein superabsorbierendes Material aus der Speicherschicht kann beispielsweise allein durch den Prozess mit Schwerkraftunterstützung in die untere Verteilungsschicht gerüttelt werden. Diese Lage kann beispielsweise durch eine anschließende Wärme- und/oder Druckbehandlung oder eine sonstige Verdichtung fixiert werden.

Eine Weiterbildung sieht ein Verfahren zur Herstellung einer absorbierenden Struktur vor, welches mindestens die folgenden Schritte umfasst:
- Ablegen einer ersten Airlaid-Lage zur Ausbildung einer Flüssigkeitsaufnahmeschicht, die vorzugsweise zumindest Cellulosefasern und Bikomponentenfasern umfasst,
- Ablegen einer zweiten Airlaid-Lage zur Ausbildung einer Flüssigkeitsspeicherschicht, die zumindest Cellulosefasern und ein SAP-Material, vorzugsweise SAP-Partikel und/oder SAP-Fasern umfasst,
- Ablegen einer dritten Airlaid-Lage zur Ausbildung einer Flüssigkeitsverteilungsschicht,
- vorzugsweise Aufbringen einer Bindemittelschicht, vorzugsweise einer Latexschicht auf die so erhaltenen Lagenstruktur,
- vorzugsweise Durchführen der Lagenstruktur durch ein Heizmittel, um die Lagenstruktur zu binden,
- Zuführen der Lagenstruktur zu einem Kalander, umfassend mindestens eine erste Glattwalze und eine Gegenwalze mit vorzugsweise Erhebungen, die einen Kalanderspalt bilden und
- Verdichtung der Lagenstruktur im Kalanderspalt, wobei SAP-Partikel und/oder SAP-Fasern aus der Flüssigkeitsspeicherschicht in die Flüssigkeitsverteilungsschicht hineinragen und einen flüssigkeitsabführenden Kontakt zwischen Flüssigkeitsspeicherschicht und Flüssigkeitsverteilungsschicht bilden.

Bevorzugt ist beispielsweise vorgesehen, dass zwischen zwei aufeinanderfolgenden Formierköpfen zur Erstellung einer jeweiligen Airlaidschicht eine SAP-Zuführung vorgesehen ist. Auf diese Weise kann das Hineinragen des SAP-Materials in die benachbarte Schicht besonders gesteuert werden. So wird zum Beispiel mittels des einen Formierkopfs die Flüssigkeitsspeicherschicht und mittels des anderen die Flüssigkeitsverteilungsschicht gebildet. Auch besteht auf diese Weise die Möglichkeit, dass bei der Erstellung der Flüssigkeitsspeicherschicht ein anderes SAP-Material eingesetzt wird als es zwischen den beiden Schichten zum Einsatz gelangt. Es besteht aber ebenfalls die Möglichkeit, dass beide SAP-Materialien gleich sind.

Die Schritte können in der angegebenen Reihenfolge oder aber in einer anderen Reihenfolge ausgeführt werden.

Eine Weiterbildung sieht vor, dass eine Walzenanordnung, die bei dem Verfahren eingesetzt wird, einen Druck eingestellt aufweist, mittels dem eine Verschiebung und ein Eindringen der durch die zweite Airlaid-Formungseinrichtung abgelegten SAP-Partikel und/oder SAP-Fasern in benachbarte Cellulosefaserbereiche erfolgt. Insbesondere können diese in einen benachbarten Bereich einer anderen Schicht eingedrückt werden.

Gemäß einer Ausgestaltung kann der Kalander zwei Glattwalzen aufweisen. Der Kalander kann aber auch eine Glattwalze wie auch eine Walze mit Prägeerhebungen aufweisen. Des Weiteren besteht die Möglichkeit, dass beispielsweise mittels einer Glattwalze zusätzlich Wärme aufgebracht wird, wodurch auf die Lagenstruktur eine Glättung der Oberfläche ermöglicht wird. Beispielsweise kann auf diese Weise eine Offenporigkeit einer Oberfläche des Materials verändert werden, insbesondere verringert werden. Eine Weiter Möglichkeit einer Bondierung geht zum Beispiel aus der DE 102 18 259 A1 hervor, auf die diesbezüglich im Rahmen der Offenbarung verwiesen wird..

Eine Verbindung von Airlaid-Lagen untereinander wie auch von Fasern in einer einzelnen Airlaid-Lage kann über ein Aufschmelzen der Bindemittelschicht und/oder der SAP-Partikel und/oder SAP-Fasern erfolgen. Andere Bindemittel wie beispielsweise Latex, das beispielsweise aufgesprüht wird, sind ebenfalls einsetzbar. Auch können Klebemittel genutzt werden, die beispielsweise aufgesprüht werden, zum Beispiel mittels eines Düsensprühsystems. Ein Bindemittelauftrag kann vollflächig wie auch nichtvollflächig erfolgen, zum Beispiel in Form eines regelmäßigen oder aber in Form eines unregelmäßigen Musters. Ebenfalls kann beispielsweise ein Film genutzt werden, der bei Erhitzung der Lagen zerschmilzt und dabei die Schichten und/oder die Fasern miteinander verbindet.

Eine Weiterbildung sieht vor, dass bei dem Verfahren zusätzlich eine dritte Airlaid-Lage auf der zweiten Airlaid-Lage zur Ausbildung einer Flüssigkeitsaufnahmeschicht vorgesehen ist, die zumindest Cellulosefasern und Bikomponentenfasern umfasst. Es ist beispielsweise ebenfalls vorgesehen, dass die folgenden Schritte während einer Ausübung des Verfahrens an gewünschten Stellen ausgeführt werden:
- das Aufbringen einer Latexschicht auf die Flüssigkeitsverteilungsschicht,
- die Durchführen der so erhaltenen Lagenstruktur durch ein Heizmittel, um die Bikomponentenfasern zu aktivieren und die Cellulosefasern zu binden,
- die Zuführen der Lagenstruktur zu einem Kalander und
- die Verdichtung der Lagenstruktur im Kalanderspalt.

Es hat sich als vorteilhaft erwiesen, dass die absorbierende Struktur in-line hergestellt wird. Hierdurch kann es zur Aufhebung der Grenzen zwischen den einzelnen Lagen der absorbierenden Struktur und zur Vermischung von Fasern jeweils benachbarter Schichten kommen.

Daneben ist aber auch die Herstellung der absorbierenden Struktur durch Nutzung separate Herstellungsschritte möglich. Beispielsweise können
- einzelne Schichten separat gefertigt werden und in einem nachgeschalteten Bindeprozess zu einer Lagenstruktur, die die absorbierende Struktur bildet, gebunden werden,
- Vorlaminate, Prepregs und dergleichen aus einer oder mehreren Lagen bereitgestellt werden, die anschließend mit weiteren Lagen zu einer absorbierenden Struktur ausgebildet werden.

Dabei können die Vorlaminate oder Prepregs Bestandteile einer oder mehrerer Funktionsschichten beinhalten. Unter einer Funktionsschicht wird beispielsweise eine Flüssigkeitsverteilungsschicht oder eine Flüssigkeitsspeicherschicht oder eine Flüssigkeitsaufnahmeschicht oder Bestandteile dieser, wie beispielsweise eine Teil-Schicht oder eine Binderschicht oder eine SAP-Partikel-und/oder SAP-Fasern-Schicht oder eine Schicht, umfassend Bikomponentenfasern verstanden..

Gemäß einem weiteren Gedanken der Erfindung wird eine Verwendung einer Vorrichtung zur Herstellung einer oben beschriebenen absorbierenden Struktur und/oder zur Durchführung eines oben beschriebenen Verfahrens vorgeschlagen, die zumindest die folgenden Komponenten aufweist:
ein Siebband zur Ablage von Airlaid-Lagen zur Ausbildung einer Lagenstruktur,
eine erste Airlaid-Formungseinrichtung, über welchen zumindest Cellulosefasern abziehbar sind, die eine Airlaid-Lage bilden,
eine zweite Airlaid-Formungseinrichtung, über welche zumindest Cellulosefasern und SAP-Partikel und/oder SAP-Fasern auf der Airlaid-Lage ablegbar sind und vorzugsweise eine zweite Airlaid-Lage bilden,
eine Ablagevorrichtung für eine weitere Lage;
ein Auftragsmittel, über das ein Bindemittel, vorzugsweise eine Latexschicht, aufbringbar ist,
ein Heizmittel, in welchem Bikomponentenfasern und/oder das Bindemittel aktivierbar sind,
eine Walzenanordnung, vorzugsweise ein Kalander, über welche die Lagenstruktur verdichtbar ist,
und eine Zuführeinrichtung von SAP-Partikeln und/oder SAP-Fasern, die zumindest dosiert und bevorzugt auch positionskontrolliert unter teilweisem Eindringen in die Cellulosefasern einer Airlaid-Lage zugeführt werden, die benachbart zu Cellulosefasern der zweiten Airlaid-Formungseinrichtung sind.

Die Reihenfolge der Komponenten kann von der obigen Reihenfolge abweichen. Insbesondere wird mit einer derartigen Vorrichtung eine absorbierende Struktur hergestellt, wie sie oben und noch nachfolgend weiter beschrieben wird.

Weiterhin ist vorzugsweise eine Einrichtung zur Bereitstellung von Bikomponentenfasern, die gemeinsam mit den Cellulosefasern auf dem Siebband ablegbar sind und eine Airlaid-Lage bilden, vorgesehen. Auch kann eine dritte Airlaid-Formungseinrichtung, über welche zumindest Cellulosefasern auf dem Siebband ablegbar sind, in die Anlage integriert werden. Beispielsweise kann die absorbierende Struktur drei Airlaidlagen aufweisen, die miteinander verbunden sind.

Eine Ausführung sieht eine Weiterbildung der Vorrichtung zur Herstellung einer absorbierenden Struktur vor, aufweisend zumindest:
- ein Siebband zur Ablage von Airlaid-Lagen zur Ausbildung einer Lagenstruktur,
- eine erste Airlaid-Formungseinrichtung, über welchen zumindest Cellulosefasern abziehbar sind und vorzugsweise eine Einrichtung zur Bereitstellung von Bikomponentenfasern, die gemeinsam mit den Cellulosefasern auf dem Siebband ablegbar sind und eine erste Airlaid-Lage bilden,
- eine zweite Airlaid-Formungseinrichtung, über welche zumindest Cellulosefasern und SAP-Partikel und/oder SAP-Fasern auf der ersten Airlaid-Lage ablegbar sind und vorzugsweise eine zweite Airlaid-Lage, bilden, wobei die zweite Airlaid-Formungseinrichtung SAP-Partikel dosiert und positionskontrolliert zugibt,
- eine dritte Airlaid-Formungseinrichtung, über welche zumindest Cellulosefasern auf dem Siebband ablegbar sind,
- ein Auftragsmittel, über das ein Bindermittel, vorzugsweise eine Latexschicht auf die Lagenstruktur aufbringbar ist,
- ein Heizmittel, in welchem Bikomponentenfasern und/oder das Bindemittel aktivierbar sind,
- eine Walzenanordnung, vorzugsweise ein Kalander, über welche die Lagenstruktur verdichtbar ist und die einen Druck einstellbar aufweist, mittels dem eine Verschiebung und Eindringen der durch die zweite Airlaid-Formungseinrichtung abgelegten SAP-Partikel in benachbarte Cellulosefaserfaserbereiche ermöglicht ist.

Eine weitere Ausgestaltung sieht vor, dass zwischen zwei Formierköpfen, bevorzugt einem zweiten und einem dritten Formierkopf zur Herstellung einer jeweiligen Airlaidschicht, eine Zuführung vorgesehen ist, mittels der zumindest ein superabsorbierendes Material, bevorzugt SAP-Partikel und/oder SAP-Fasern, zugeführt werden kann. Eine derartige Zuführung kann auch an einem anderen Orte entlang der Vorrichtung vorgesehen sein, zusätzlich oder alternativ.

Eine Dosierung der SAP-Partikel und/oder SAP-Fasern kann sich über eine Breite des Materials unterscheiden. Auch besteht die Möglichkeit, unterschiedliche SAP-Materialen über die Breite des Materials an verschiedenen Orten wie auch an gleichen Orten anzuordnen, insbesondere abzulegen. Eine Ausgestaltung sieht vor, dass über eine Dicke des Materials SAP-Partikel in einer Schicht unterschiedlich angeordnet werden. Eine Positionskontrolle erfolgt beispielsweise über eine gezielte Ausrichtung der SAP-Zuführung. Auch besteht die Möglichkeit, diese Positionskontrolle automatisch auszuführen, zum Beispiel über Sensoren, bildanalysierende Verarbeitung oder ähnliches. Auch besteht die Möglichkeit, dass die Lage der SAP-Partikel und/oder SAP-Fasern in der Schicht automatisch geprüft wird, zum Beispiel mittels Detektierung der SAP-Partikel und/oder SAP-Fasern. Dazu können SAP-Partikel und/oder SAP-Fasern beispielsweise eine detektierbare Kennzeichnung, zum Beispiel ein spezielles Material, ein Farbe oder anderes aufweisen. Dieses erlaubt beispielsweise eine Korrektur während des laufenden Herstellungsverfahrens.

Eine Weiterbildung sieht eine Vorrichtung vor, wobei in einem Kalander neben einer ersten Glattwalze und einer Gegenwalze mit Erhebungen, die einen ersten Kalanderspalt bilden, eine zweite Glattwalze vorgesehen ist, die derart zur Gegenwalze angeordnet ist, dass ein zweiter Walzenspalt gebildet wird, in dem bei Durchlauf einer absorbierenden Struktur mit einer Flüssigkeitsaufnahmeschicht die zweite Glattwalze in Kontakt mit der Flüssigkeitsaufnahmeschicht kommt. Die Glatt- und Prägewalzen sind beheizbar. Hierdurch erhält die Flüssigkeitsaufnahmeschicht eine zusätzliche Verdichtung und Glättung. In dieser Anordnung kommt die Flüssigkeitsverteilungslage der absorbierenden Struktur mit den Erhebungen der Gegenwalze in Kontakt.

Die Weiterbearbeitung der absorbierenden Strukturen kann direkt im Anschluß nach der Lagenerstellung erfolgen. Die absorbierenden Strukturen können aber noch zusammenhängend auch aufgerollt oder über eine Festooning-Einheit transportierbar gemacht werden. Eine Weiterverarbeitung kann sodann an einem anderen Ort erfolgen. Eine Weiterverarbeitung kann zum Beispiel eine Beschichtung, ein weiteres Laminieren mit ein oder mehreren anderen Schichten, ein Schneiden in Längs- und/oder Querrichtung, ein weiteres Verdichten und/oder Bondieren, ein Recken und/oder einen sonstigen Schritt umfassen.

Komponenten einer Airlaid-Herstellungsvorrichtung und deren jeweiliger Einsatz gehen zum Beispiel aus der DE 10 2004 009 556 A1 betreffend die Herstellung einer Faserbahn aus Cellulosefasern, der DE 10 2004 214 53 A1 betreffend eines Formkopfes wie auch eines Verfahrens zur Herstellung einer Airlaid-Schicht, aus der DE 10 2004 056 154 A1 betreffend eine Transportvorrichtung hervor. Weiterhin geht aus der DE 103 270 26 A1 ein Verfahren zur Herstellung eines Faservlieses nach einem Airlaid-Verfahren wie auch eine dafür geeignete Faser hervor. Aus der DE 199 183 43 A1 geht wiederum ein Airlaid-Verfahren und eine Airlaidlage hervor, bei der auch eine Bindefaser zum Einsatz gelangt. Aus der WO 2005/080655 A1 ist wiederum der Aufbau einer Airlaidanlage mit verschiednen weiteren Komponenten und deren Lageanordnung und Zweck entnehmbar. Eine Detektierung von SAP und deren kontrollierte Abgabe und eventuelle Korrektur ebenso wie die Erstellung voneinander getrennter absorbierender Strukturen geht zum Beispiel aus der WO03/034963 A2 hervor.

Die oben genannten wie auch die dort im Stand der Technik als Entgegenhaltung genannten Druckschriften geben weitere Möglichkeiten an, wie die Vorrichtung ausgestaltet sein kann. Im Rahmen der Offenbarung der Erfindung wird auf diese Druckschriften wie auch auf den in der Einführung genannten Stand der Technik vollumfänglich verwiesen.

Die absorbierende Struktur kann in
- Hygieneprodukten, wie beispielsweise Babywindeln, Damenhygiene, Inkontinenzprodukten, Abschminktücher,
- Medikalprodukten, wie beispielsweise OP-Abdeckungen und
- industriellen Produkten, wie beispielsweise Abdeckmatten, Wischtüchern, und
- Lebensmittelschalen zum Flüssigkeitsaufsaugen
eingesetzt werden. Dazu kann die absorbierende Struktur selbst zumindest eine Außenfläche, bevorzugt beide Außenflächen eines Produkts bilden. Die absorbierende Struktur kann aber auch zumindest an einer Seite, beispielsweise auch an jeder Seite mit einer zusätzlichen Lage zumindest abgedeckt, bevorzugt verbunden sein.

Weitere vorteilhafte Ausgestaltungen und Weiterbildungen der vorliegenden Erfindung werden anhand der nachfolgenden Beispiele, die auch in den Zeichnungen dargestellt sind, näher erläutert. Die dabei dargelegten Merkmale sind nicht auf die einzelne Ausgestaltung beschränkt sondern können untereinander wie auch mit den weiter oben beschriebenen Merkmalen zu zusätzlichen Weiterbildungen verknüpft werden, die im einzelnen jedoch hier nicht ausgeführt werden. Es zeigen:
Fig. 1 bis Fig. 4 schematische Ansichten von Querschnitten verschiedener bondierter absorbierender Strukturen unter Verwendung von Airlaid-Vliesen,
Fig. 5 eine schematische Ansicht einer ersten möglichen Ausgestaltung einer Herstellungsvorrichtung, und

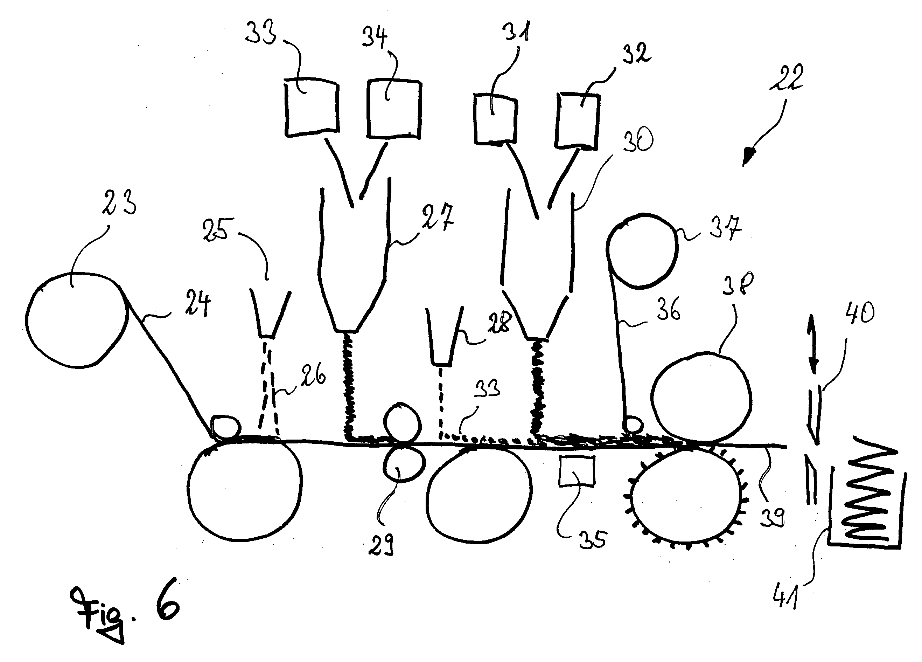

Fig. 6 eine schematische Ansicht einer zweiten möglichen Ausgestaltung einer Herstellungsvorrichtung.

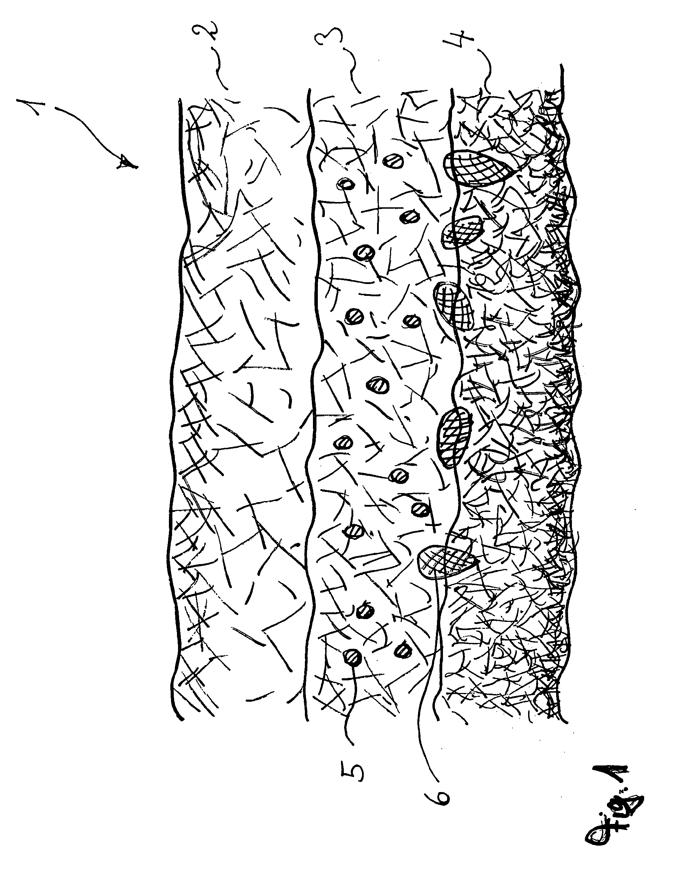

Die in Figur 1 dargestellt absorbierende Struktur 1 wurde aus drei Lagen eines Airlaid-Materials gefertigt, die übereinander angeordnet sind und in einem anschließenden Kalandrierprozess unter Anwendung von Wärme und Druck miteinander verbunden worden sind. Die Lagen-Struktur weist folgende Schichten auf:
- eine Flüssigkeitsaufnahmeschicht 2 aus Cellulose- und Bikomponentenfasern,
- eine Flüssigkeitsspeicherschicht 3 aus Cellulosefasern und mit ersten 5 und zweiten 6 SAP-Partikeln, sowie
- eine Flüssigkeitsverteilungsschicht 4 aus Cellulosefasern.

Durch die Art des Ablegeprozesses für die Airlaid-Lagen und des Bindeprozesses, der für die Herstellung der absorbierenden Struktur genutzt worden ist und der in diesem Falle ein in-line-Prozess ist, sind die einzelnen Airlaid-Lagen der Flüssigkeitsverteilungsschicht 4, der Flüssigkeitsspeicherschicht 3 und der Flüssigkeitsaufnahmeschicht 2 innerhalb des Grenzbereiches nicht ineinander übergegangen, wobei die Fasern benachbarter Bereiche anhand eines identifizierbaren Grenzverlaufs voneinander trennbar sind. Dieser Grenzverlauf kann beispielsweise mittels farblicher Unterscheidung der Fasern verschiedener Schichten deutlich hervorgehoben werden. Die Airlaid-Lagen sind innerhalb der Lagenstruktur gut voneinander unterscheidbar. In den beiden äußeren Randschichten ist beispielsweise eine stärkere Verdichtung der Fasern erkennbar. Die ersten und die zweiten SAP-Partikel 5, 6 sind zufällig verteilt in der absorbierenden Struktur angeordnet und sind zumindest punktuell mit den Fasern verklebt. Im mittleren Bereich der absorbierenden Struktur sind wenige kleine Kavitäten erkennbar, in denen weder Fasern noch SAP-Partikel angeordnet sind.

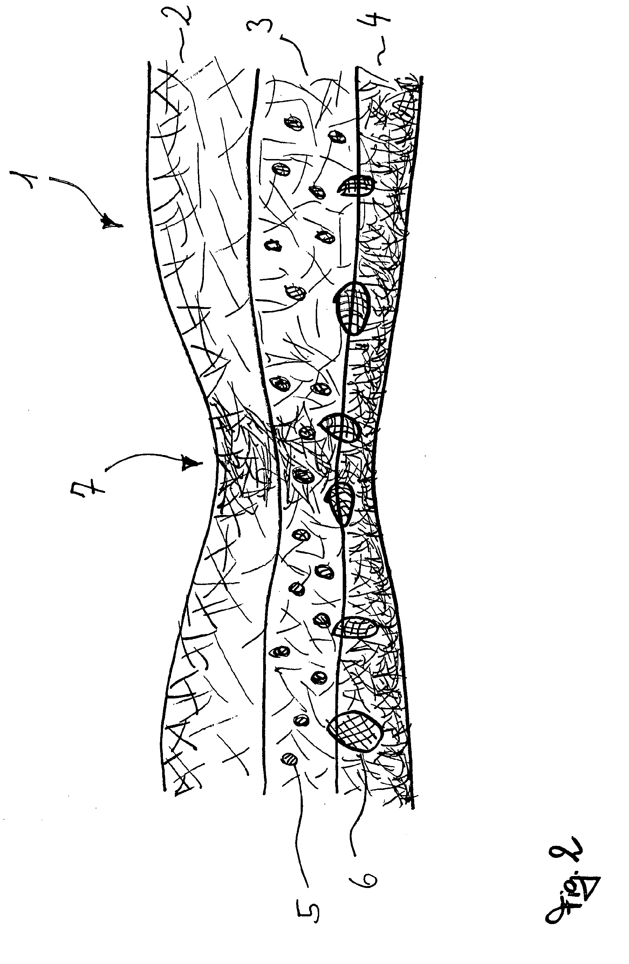

In Figur 2 ist eine Lagen-Struktur dargestellt, die eine andere Stelle des gleichen Materials wie gemäß Fig. 1 in beispielhafter schematischer Ansicht wiedergibt. Deutlich erkennbar ist ein Bindepunkt 7, in dem eine wesentlich stärkere Verdichtung des Materials erfolgt ist. Die Airlaid-Lagen sind innerhalb der Lagenstruktur in beiden Figuren weiterhin voneinander unterscheidbar. Erkennbar ist in Figur 2, dass bevorzugt auch an den beiden äußeren Randschichten der absorbierenden Struktur 1 eine stärkere Verdichtung der Fasern erfolgt ist als in einem mittleren Bereich.

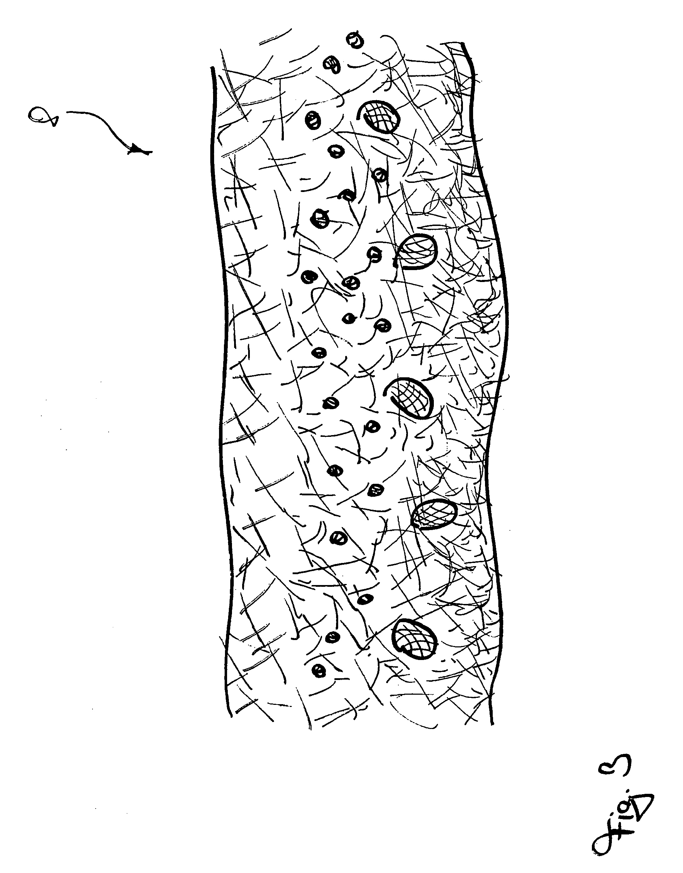

Die Figur 3 zeigt eine zweite Lagen-Struktur 8 ohne die oben genannten Unterschiede zwischen den Randbereichen und dem jeweiligen mittleren Bereich der absorbierenden Struktur. Nur ganz schwach sowohl in den mittleren als auch in den Randbereichen der absorbierenden Struktur sind vereinzelte stärkere Verdichtungen, aber auch vereinzelte Kavitäten sichtbar. Die Schichten gehen aber ineinander über; besitzen keine klare Grenze, sondern einen Mischbereich.

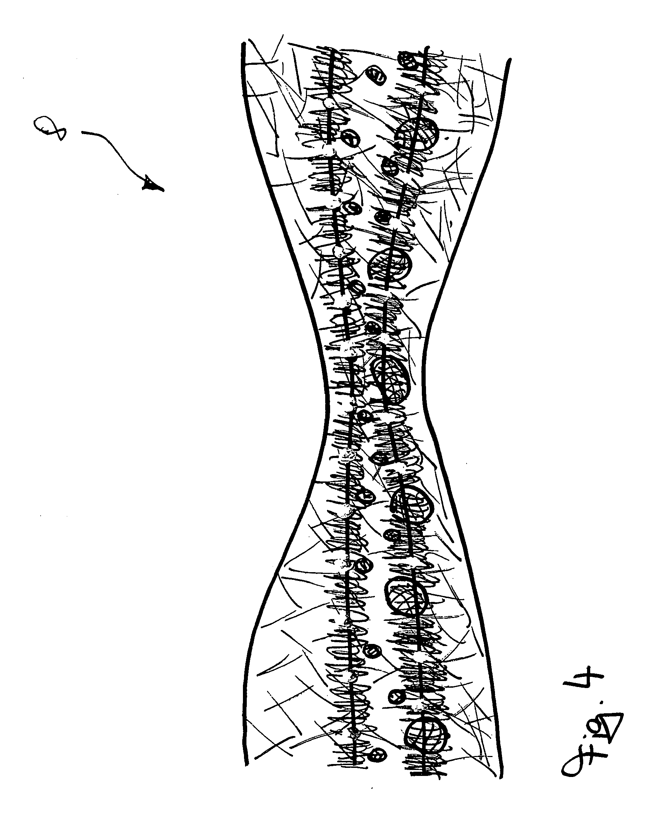

Diese Tendenz der Vereinheitlichung der Lagen während des Ablege- und Bindeprozesses ist noch stärker in der Figur 4 erkennbar, die ebenfalls das Material gemäß Figur 3 aufweist. Die absorbierende Struktur 8 weist eine fast einheitliche Verdichtung der Fasern auf, insbesondere durch die Verdichtung, wie sie in einem Bondierungsbereich erfolgt ist. Die einzelnen Airlaid-Lagen sind nicht mehr erkennbar. Selbst in Bereichen, in denen SAP-Partikel angeordnet sind, die im Sinne von Störstellen während der Lagen- und Verbundbildung angesehen werden könnten, sind keine Unterschiede in Bezug auf eine Verdichtung oder Anordnung von Kavitäten erkennbar. Im mittleren bis unteren Bereich der absorbierenden Struktur sind SAP-Partikel zufällig verteilt angeordnet. Das Material weist eine im Wesentlichen gleichmäßige Verdichtung auf. Dieser Effekt kann erzeugt werden, wenn zur Herstellung einer absorbierenden Struktur Lagen verwendet werden, die im Wesentlichen das gleiche Material und vorzugsweise auch eine ähnliche Materialdicke umfassen. Beispielsweise nach dem Bindeprozess ist die Dicke der nicht mehr voneinander unterscheidbaren Lagen im Wesentlichen jeweils gleich. Unter dieser Voraussetzung und der Tatsache, dass die SAP-Partikel verstärkt im unteren Teil der absorbierenden Struktur erkennbar sind, ragen SAP-Partikel aus der Flüssigkeitsspeicherschicht in die Flüssigkeitsverteilungsschicht hinein. Durch Anfärbung kann dies auch kenntlich gemacht werden.

Des Weiteren geht aus Fig. 4 die Möglichkeit hervor, dass eine Mischzone vorhanden sein kann. Diese Mischzone ist durch eine gestrichelte Linie als angedeutete Mittellinie zwichen zwei Schichten eingezeichnet, wobei die Fasern der einen Schicht sich mit denen der anderen Schicht in den Abschnitten miteinander vermengen, die durch senkrecht zu der Mittellinie verlaufende Striche hervorgehoben sind.

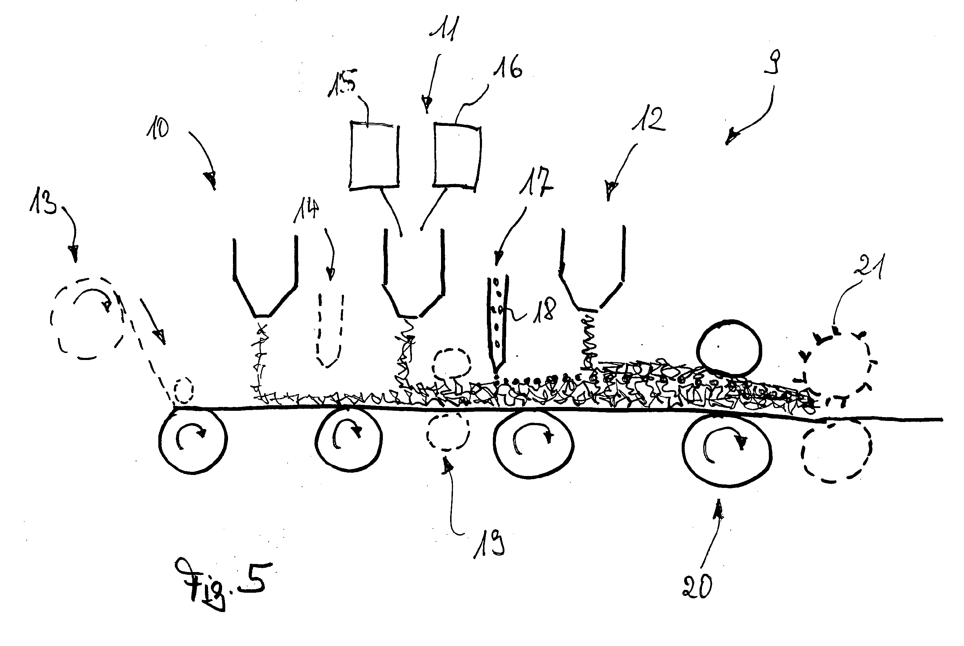

Fig. 5 zeigt in schematischer Ansicht eine mögliche Ausgestaltung einer Vorrichtung 9 zur Herstellung einer absorbierenden Struktur umfassend drei Airlaid-Schichten. Die Vorrichtung 9 ist dargestellt als In-Line-Prozeß mit einer ersten, einer zweiten und einer dritten Airlaid-Formungseinrichtung 10, 11, 12. Beispielhaft ist ebenfalls dargestellt, dass eine Airlaidschicht auch vorgefertigt wird. Eine Abrollkalander 13 dafür ist gestrichelt dargestellt. Es können auch zwei oder alle Airlaid-Lagen vorgefertigt sein und erst anschließend zusammengefügt werden. Zwischen der ersten und der zweiten Airlaid-Formungseinrichtung 10,11 kann beispielsweise eine erste Zuführung 14 angeordnet sein. Diese ist gestrichelt angedeutet. Mittels dieser kann zum Beispiel ein SAP-Material und/oder ein Bindematerial zugeführt werden, bevorzugt zwischen zwei Airlaid-Schichten. Exemplarisch ist dargestellt, dass die zweite Airlaid-Formungseinrichtung 11 ein erstes und ein zweites Material 15, 16 miteinander vermischt, bevor das Gemisch beider Materialien 15,16 auf dem Siebband ebenfalls abgelegt wird. Ein derartiges Gemisch kann zum Beispiel Cellulosefasern mit Bindefasern, Cellulosefasern mit SAP-Fasern und/oder Partikeln wie auch andere Kombinationen an Mischungen ermöglichen. Eine derartige Mischung ist nicht nur mittels der zweiten Airlaid-Formungseinrichtung 11 ermöglicht. Vielmehr kann auch die erste und/oder die dritte Airlaid-Formungseinrichtung 10, 12 derartiges vorsehen. Zwischen der zweiten und der dritten Airlaid-Formungseinrichtung 11, 12 ist eine zweite Zuführung 17 angeordnet. Dort wird zum Beispiel das superabsorbierende Material 18 zugeführt, was schließlich von der Flüssigkeitsspeicherschicht in die Flüssigkeitsverteilungsschicht hinragt. Der zweiten Formungseinrichtung 11 nachgelagert kann zum Beispiel eine Heizeinrichtung 19 angeordnet sein. Die Heizeinrichtung 19 ist hier als Glatt-Walzenkalander dargestellt. Es kann aber auch eine Infrarot-Heizung, ein Ofenabschnitt oder eine sonstige Beheizung vorgesehen sein. Damit kann zum Beispiel eine Bindefaser aktiviert werden, so dass die Fasern beider Schichten untereinander aber auch jeweils miteinander sich verbinden. Durch die Nutzung es Glattwalzenkalanders kann des Weiteren ein Druck auf das Zwischenmaterial und damit ein erstes Verdichten ausgeübt werden. Das über die zweite Zuführung 18 zugeführte Material wird sodann durch Fasern abgedeckt, die aus der dritten Airlaid-Formungseinrichtung 12 abgelegt werden. Im Anschluss daran kann sich wieder eine Verfestigung anschließen, zum Beispiel wie dargestellt mittels eines beheizten Kalanders 20 als Verfestigungseinheit. Eine Verfestigungseinheit kann aber zum Beispiel auch eine Verdichtung mittels Hydrojets vorsehen. Eine weitere Verfestigung ergibt sich zum Beispiel durch den Einsatz eines Kalanders mit Prägungen 21. Hierdurch kann der Lagenstruktur eine weitere Oberflächeneigenschaft aufgeprägt werden, zum Beispiel ein Muster, eine Einstellung einer offen gebliebenen Fläche, eine Verdrängung von SAP-Material von einer in die nächste benachbarte Schicht. Des Weiteren können auch Komponenten in der Vorrichtung wie auch ein grundsätzlicher Aufbau Verwendung finden, wie sie aus der WO 00/74620 hervorgehen und auf die im Umfang der Offenbarung vollständig verwiesen wird.

Fig. 7 zeigt eine zweite mögliche prinzipielle Ausgestaltung einer Herstellungsvorrichtung 22. Von einem Wickler 23 wird ein Tissue 24, d.h. ein nassabgelegtes Fasermaterial, einer Weiterverarbeitung zugeführt. Das Tissue kann zum Beispiel über eine Bindemitteleinrichtung 25 mit einem Bindemittel 26 versehen sein. Das Bindemittel 26 kann eine Bindefaser umfassen, einen Latexsprühauftrag und/oder andere zum Verbinden von Fasern geeignete Mittel. Die Bindemitteleinrichtung 25 muss auch nicht an diesem Orte angeordnet sein. Vielmehr kann die Bindeeinrichtung auch erst nach beispielsweise einer Airlaid-Ablegeeinheit 27 und einer SAP-Zuführungseinheit 28 zum Auftragen von erstem SAP angeordnet werden, wenn diese ihre jeweiligen Materialien auf das Tissue 24 aufgeschichtet haben. Das Bindemittel 26 kann dann dazu dienen, das SAP in der Lage zu stabilisieren, insbesondere zu fixieren. Das Bindemittel kann auch dazu genutzt werden, eine Porengröße zu ändern, insbesondere diese zu verkleinern. Bevorzugt bleibt aber trotz Bindemittelauftrag dieser Bereich luft- und flüssigkeitsdurchlässig. Partiell kann dieser auch für zumindest Flüssigkeit gesperrt sein. Bei der dargestellten Verfahrensweise wird das Airlaid auf das Tissue abgelegt und mittels einer anschließenden Kalandrierung unter Hitze und Druck verdichtet und verbunden. Der Kalander 29 kann hierzu beheizt und hinsichtlich seines Spaltmaßes unterschiedlich einstellbar sein. Nach der Verdichtung der so gebildeten Flüssigkeitsverteilungsschicht wird über eine zweite Airlaid-Ablegeeinheit 30 ein weiteres Airlaifmaterial abgelegt. Die Cellulosefasern werden hierbei mit zweitem SAP 32 vermischt und sodann gemeinsam abgelegt. Vorab kann, braucht aber nicht die SAP-Zuführungseinheit erstes SAP 33 direkt auf die Flüssigkeitsverteilungsschicht abzulegen. Des weiteren kann auch die Flüssigkeitsverteilungsschicht neben Cellulosefasern ebenfalls ein SAP-Material 34 beinhalten. Der Gewichtsanteil an ersten und zweitem SAP zusammen ist aber höher als der Gewichtsanteil des SAP-Materials 34. Durch die Bewegung beim Herstellungsprozess, insbesondere durch eine Rüttelmaschine 35, die beispielsweise direkt auf das Laufband einwirkt, kann ein Wandern von SAP in die Flüssigkeitsverteilungsschicht hervorgerufen werden. Die Rüttelmaschine 35 kann dazu zum Beispiel mit einer einstellbaren Frequenz angepasst an die Bandgeschwindigkeit ein Wandern beeinflussen. Ein Rütteln und dadurch hervorgerufenes Wandern kann ebenfalls durch eine Bewegung in der Herstellungsvorrichtung selbst hervorgerufen werden, beispielsweise durch eine bewusst ungedämpftes Weitergeben von Stößen. Auch kann beispielsweise die Bandbewegung gezielt ausgenutzt werden, um ein Wandern des SAP zu initiieren. Das Tissue hat bevorzugt eine Porengröße, die kleiner als die SAP-Größe ist. Immigriert daher SAP in die Flüssigkeitsverteilungsschicht hinein oder ist eventuell selbst dort schon vorhanden, verhindert das Tissue ein Austreten und Beeinträchtigen des Siebbandes, auf dem die Schichten transportiert werden. Eine Flüssigkeitsaufnahmeschicht 36 wird zum Beispiel vorgefertigt von einem Wickler 37 abgespult und zugeführt. Bevorzugt weist die Flüssigkeitsaufnahmeschicht thermoplastische Fasern auf, insbesondere besteht die Flüssigkeitsaufnahmeschicht aus thermoplastischen Fasern. Ein weitere Kalander 38 verdichtet und verbindet die Schichten weiter miteinander mittels Druck und Temperatur. Eine Profilierung, insbesondere die Anordnung von erhöhten Bondingbereichen auf einer der Walzen des Kalanders, erfolgt bevorzugt auf der der Flüssigkeitsverteilungsschicht zugekehrten Seite. Ein gegenüberliegende bevorzugte Glattwalze ist dagegen bevorzugt der Flüssigkeitsaufnahmeschicht zugekehrt. Durch das Kalandrieren kann ein weiteres Verschieben bzw. Eindringen von SAP aus der Flüssigkeitsspeicherschicht in die Flüssigkeitsverteilungsschicht erfolgen. Eine Weiterbehandlung der so gebildeten absorbierenden Struktur 39 kann direkt im Anschluss erfolgen, zum Beispiel mittels einer Schneideeinheit 40, die bevorzugt in Längs- und Querrichtung die aneinanderhängenden über die Breite verteilten einzelnen absorbierenden Strukturen voneinander trennt bzw. diese transportfähig macht, zum Beispiel mittels einer Festooningeinheit 41. Eine Herstellungsvorrichtung kann beispielsweise auch vorsehen, dass neben einer Zumischung von SAP andere Materialien zugeführt werden, zum Beispiel odorbeeinflussende Materialien, hydrophile oder hydrophobe Ausrüstungen, ebenso Farben, Indikatoren, Flammhemmer, Folien oder sonstiges. Auch kann eine Fertigung von absorbierenden Strukturen erfolgen, wie sie beispielsweise aus der WO95/03019A1, der WO 03/000163A1 hervorgeht und auf die insgesamt, aber auch bezüglich der Streifenform und des Schneidens verwiesen wird.

## Patentansprüche

1. Absorbierende Struktur mit einer Folge an Schichten umfassend mindestens eine Flüssigkeitsaufnahmeschicht (2), eine nachfolgende Flüssigkeitsspeicherschicht (3) mit superabsorbierendem Polymer SAP, vorzugsweise SAP-Partikel und/oder SAP-Fasern, und eine nachfolgende Flüssigkeitsverteilungsschicht (4), wobei die Schichten verbunden und eine Lagenstruktur bilden, wobei zumindest die Flüssigkeitsspeicherschicht (3) und die Flüssigkeitsverteilungsschicht (4) zumindest jeweils ein Airlaid-Material, vorzugsweise eine Airlaid-Lage umfassend Cellulosefasern als Hauptbestandteil aufweisen, ein Bereich des Airlaid-Materials in der Flüssigkeitsverteilungsschicht (4) engporiger ist als ein Bereich des Airlaid-Materials in der Flüssigkeitsspeicherschicht (3), und wobei die Flüssigkeitsspeicherschicht (3) superabsorbierendes Polymer, insbesondere SAP-Partikel aufweist, das aus der Flüssigkeitsspeicherschicht (3) In die Flüssigkeitsverteilungsschicht (4) hineinragt zur Erzeugung eines Rücksaugeffekts für Flüssigkeit, die durch die Flüssigkeitsaufnahmeschicht (2) und Flüssigkeitespeicherschicht (3) in die Flüssigkeitsverteilungsschicht (4) eingetreten ist, wobei mehr als 20% der In der Flüssigkeitsspeicherschicht (3) angeordneten SAP-Partikel und/oder SAP-Fasern in die Flüssigkeitsverteilungsschicht (4) hineinragen.

2. Absorbierende Struktur nach Anspruch 1, **dadurch gekennzeichnet, dass** die Flüssigkeitsaufnahmeschicht (2) ein Airlaid-Material aufweist.

3. Absorbierende Struktur nach Anspruch 1 oder 2, **dadurch gekennzeichnet, dass** die Flüssigkeitsaufnahmeschicht (2) thermoplastische Fasern aufweist.

4. Absorbierende Struktur nach Anspruch 3, **dadurch gekennzeichnet, dass** die Flüssigkeitsaufnahmeschicht (2) ein voluminöses Vlies aus thermoplastischen Fasern aufweist.

5. Absorbierende Struktur nach einem der vorhergehenden Ansprüche, **dadurch gekennzeichnet, dass** das superabsorbierende Polymer unter Verdrängung von Fasern der Flüssigkeitsverteilungsschicht (4) In diese aus der Flüssigkeitsspeicherschicht (3) hineinragt.

6. Absorbierende Struktur nach einem der vorhergehenden Ansprüche 1 bis 4, **dadurch gekennzeichnet, dass** das superabsorbierende Polymer, bevorzugt SAP-Partikel, unregelmäßig in die Flüssigkeitsverteilungsschicht (4) aus der Flüssigkeitsspeicherschicht (3) hineinragt

7. Absorbierende Struktur nach einem der vorhergehenden Ansprüche, **dadurch gekennzeichnet, dass** SAP-Partikel und/oder SAP-Fasern zumindest zu einem Drittel ihrer Längserstreckung in die Flüssigkeitsepeicherschicht (3) hineinragen.

8. Absorbierende Struktur nach einem der vorhergehenden Ansprüche, **dadurch gekennzeichnet, dass** mehr als 40% der in der Flüssigkeitsspeicherschicht (3) angeordneten SAP-Partikel und/oder SAP-Fasern in die Flüssigkeitsverteilungsschicht (4) hineinragen.

9. Absorbierende Struktur nach einem der vorhergehenden Ansprüche, **dadurch gekennzeichnet, dass** mehr als 40% aber weniger als 70% der in der Flüssigkeitsspelcherschlcht (3) angeordneten SAP-Partikel und/oder SAP-Fasern in die Flüssigkeitsvertellungsschicht (4) hineinragen.

10. Absorbierende Struktur nach einem der vorhergehenden Ansprüche, **dadurch gekennzeichnet, dass** das in die Flüssigkeitsverteilungsschicht (4) hineinragende SAP-Materlal nach Flüssigkeitsaufnahme sich mehr in der Flüssigkeitsspeicherschicht (3) denn in der Flüssigkeitsverteilungsschicht (4) vergrößert hat.

11. Absorbierende Struktur nach einem der vorhergehenden Ansprüche, **dadurch gekennzeichnet, dass** in die Flüssigkeitsverteilungsschicht (4) hineinragende SAP-Partikel und/oder SAP-Fasern nach Flüssigkeitsaufnahme sich in die Flüssigkeitsverteilungsschicht (4) zumindest teilweise zurückgezogen haben.

12. Absorbierende Struktur nach einem der vorhergehenden Ansprüche, **dadurch gekennzeichnet, dass** die Lagenstruktur innerhalb der Flüssigkeitsverteilungsschicht (4) und/oder der Flüssigkeitsspeicherschicht (3) und/oder der Flüssigkeitsaufnahmeschicht (2) und/oder in einem Grenzbereich zwischen der Flüssigkeitsverteilungsschicht (4) und/oder der Flüssigkeitsspelcherschicht (3) und/oder der Flüssigkeitsaufnahmeschicht (2) Kavitäten aufweist.

13. Absorbierende Struktur nach einem der vorhergehenden Ansprüche, **dadurch gekennzeichnet, dass** Kavitäten in der Flüssigkeitsspeicherschicht (3) größer ausgebildet sind als in der Flüssigkeitsaufnahmeschicht (2) und/oder in der Flüssigkeitsverteilungsschicht (4).

14. Absorbierende Struktur nach einem der vorhergehenden Ansprüche, **dadurch gekennzeichnet, dass** zumindest ein Teil der Cellulosefasern der Airlaid-Lagen der Flüssigkeitsverteilungsschicht (4) und/oder der Flüssigkeitsspeicherschicht (3) und/oder der Flüssigkeitsaufnahmeschicht (2) in einem jeweils dazwischen liegenden Grenzbereich miteinander vermischt ist.

15. Absorbierende Struktur nach einem der vorhergehenden Ansprüche, **dadurch gekennzeichnet, dass** die Airlaid-Lagen der Flüssigkeitsverteilungsschicht (4) und/oder der Flüssigkeitsspeicherschicht (3) und/oder der Flüssigkeitsaufnahmeschicht (2) innerhalb des Grenzbereiches ineinander übergehen.

16. Absorbierende Struktur nach einem der vorhergehenden Ansprüche, **dadurch gekennzeichnet, dass** die Flüssigkeitsverteilungsschicht (4) eine erste und eine zweite Oberfläche aufweist, wobei die erste Oberfläche in Kontakt mit der Flüssigkeitsspeicherschicht (3) steht und wobei die Flüssigkeitsverteilungsschicht (4) an ihrer zweiten Oberfläche stärker verdichtet ist, als an ihrer ersten Oberfläche.

17. Verfahren zur Herstellung einer absorbierenden Struktur (1), umfassend mindestens die folgenden Schritte:
- Ablegen einer ersten Lage, bevorzugt einer Airlaid-Lage, die vorzugsweise zumindest Cellulosefasern und Bikomponentenfasern umfasst, und/oder einer hochvoluminösen Vlieslage aus thermoplastischen Fasern, zur Ausbildung einer Flüssigkeitsaufnahmeschicht (2),
- Ablegen einer zweiten Airlaid-Lage zur Ausbildung einer Flüssigkeitsspeicherschicht (3), die zumindest Cellulosefasern und SAP-Partikel und/oder SAP-Fasern umfasst,
- Ablegen einer dritten Airlaid-Lage zur Ausbildung einer Flüssigkeitsverteilungsschicht (4),
- vorzugsweise Aufbringen einer Bindemittelschicht, vorzugsweise einer Latexschicht auf eine Lage, vorzugsweise auf die so erhaltene Lagenstruktur,
- vorzugsweise Durchführen der Lagenstruktur durch ein Beizmittel, um die Lagenstruktur zu binden,
- Zuführen zumindest einer Lage, bevorzugt der Lagenstruktur zu einem Kalander, umfassend mindestens eine Glattwalze und eine Gegenwalze vorzugsweise mit Erhebungen, die einen Kalanderspalt bilden,
- Verdichtung der zumindest einen Lage, bevorzugt der Lagenstruktur im Kalanderspalt, wobei mehr als 20% der In der Flüssigkeitsspeicherschicht (3) angeordneten SAP-Partikel und/oder SAP-Fasern aus der Flüssigkeitsspeicherschicht (3) in die Flüssigkeitsvertellungsschicht (4) hineinragen und einen flüssigkeitsabführenden Kontakt zwischen Flüssigkeitsspelcherschicht (3) und Flüssigkeitsverteilungsschicht (4) bilden.

18. Verwendung einer Vorrichtung zur Herstellung einer absorbierenden Struktur (1) nach einem der Ansprüche 1 bis 16 und/oder zur Durchführung eines Verfahrens nach Anspruch 17, aufweisend zumindest
- ein Siebband zur Ablage von Airlaid-Lagen zur Ausbildung einer Lagenstruktur,
- eine erste Airlaid-Formungseinrichtung (10), über welchen zumindest Cellulosefasern abziehbar sind, die eine Airlaid-Lage bilden,
- eine zweite Airlaid-Formungseinrichtung (11), über welche zumindest Cellulosefasern und SAP-Partikel und/oder SAP-Fasern auf der Airlaid-Lage ablegbar sind und vorzugsweise eine zweite Airlaid-Lage bilden,
- eine Ablagevorrichtung für eine weitere Lage;
- ein Auftragsmittel, über das ein Bindemittel (26), vorzugsweise eine Latexschicht, aufbringbar ist,
- ein Heizmittel, in welchem Bikomponentenfasern und/oder das Bindemittel (26) aktivierbar sind,
- eine Walzenanordnung, vorzugsweise ein Kalander, über welche die Lagenstruktur verdichtbar ist,
- und eine Zuführeinrichtung (28) von SAP-Partlkeln und/oder SAP-Fasern, die zumindest dosiert und bevorzugt auch positionskontrolliert unter teilwelsem Eindringen in die Cellulosefasern einer Airlaid-Lage zugeführt werden, die benachbart zu Cellulosefasern der zweiten Airlaid-Formungseinrichtung (11) sind.

## Claims

1. An absorbent structure with a sequence of layers, comprising at least one liquid absorbent layer (2), a subsequent liquid storage layer (3) with super absorbent polymer SAP, preferably SAP particles and/or SAP fibers, and a subsequent liquid distribution layer (4), wherein the layers are connected and form a layer structure, wherein at least the liquid storage layer (3) and the liquid distribution layer (4) at least respectively include an air-laid material as a main component, preferably an air-laid layer which includes cellulose fibers, wherein a portion of the air-laid material in the liquid distribution layer (4) has smaller pores than a portion of the air-laid material in the liquid distribution layer (3), and wherein the liquid storage layer (3) includes super absorbent polymer, preferably SAP particles, which extends from the liquid storage layer (3) into liquid distribution layer (4) for generating a reverse suction effect for liquid which has entered through the liquid absorption layer (2) and the liquid storage layer (3) into the liquid distribution layer (4), wherein more than 20% of the SAP particles and/or SAP fibers arranged in the liquid storage layer (3) extend into the liquid distribution layer (4).

2. The absorbent structure according to claim 1, **characterized in that** the liquid absorption layer (2) includes an air-laid material.

3. The absorbent structure according to claim 1 or 2, **characterized in that** the liquid absorption layer (2) includes thermoplastic fibers.

4. The absorbent structure according to claim 3, **characterized in that** the liquid absorption layer (2) includes a voluminous fleece including thermoplastic fibers.

5. The absorbent structure according to any one of the preceding claims, **characterized in that** the super absorbent polymer protrudes into the liquid distribution layer (4) from the liquid storage layer (3) while displacing fibers of the liquid distribution layer (4).

6. The absorbent structure according to any one of the preceding claims 1 through 4, **characterized in that** the super absorbent polymer, preferably including SAP particles, extends into the liquid distribution layer (4) from the liquid storage layer (3) in an uneven manner.

7. The absorbent structure according to any one of the preceding claims, **characterized in that** SAP particles and/or SAP fibers at least with a third of their longitudinal extension extend into the liquid storage layer (3).

8. The absorbent structure according to any one of the preceding claims, **characterized in that** more than 40% of the SAP particles and/or SAP fibers arranged in the liquid storage layer (3) extend into the liquid distribution layer (4).

9. The absorbent structure according to any one of the preceding claims, **characterized in that** more than 40% but less than 70% of the SAP particles and/or SAP fibers arranged in the liquid storage layer (3) extend into the liquid distribution layer (4).

10. The absorbent structure according to any one of the preceding claims, **characterized in that** the SAP material extending into the liquid distribution layer (4) after liquid absorption has expanded more in the liquid storage layer (3) than in the liquid distribution layer (4).

11. The absorbent structure according to any one of the preceding claims, **characterized in that** SAP particles and/or SAP fibers extending into the liquid distribution layer (4) have at least partially retreated back into the liquid distribution layer (4) after liquid absorption.

12. The absorbent structure according to any one of the preceding claims, **characterized in that** the layer structure includes cavities within the liquid distribution layer (4) and/or the liquid storage layer (3) and/or the liquid absorption layer (2) and/or in a boundary portion between the liquid distribution layer (4) and/or the liquid storage layer (3) and/or the liquid absorption layer (2).

13. The absorbent structure according to any one of the preceding claims, **characterized in that** cavities in the liquid storage layer (3) are configured larger than in the liquid absorption layer (2) and/or the liquid distribution layer (4).

14. The absorbent structure according to any one of the preceding claims, **characterized in that** at least a portion of the cellulose fibers of the air-laid layers of the liquid distribution layer (4) and/or the liquid storage layer (3) and/or the liquid absorption layer (2) is intermixed with one another in a boundary portion respectively arranged therebetween.

15. The absorbent structure according to any one of the preceding claims, **characterized in that** air-laid layers of the liquid distribution layer (4) and/or of the liquid storage layer (3) and/or of the liquid absorption layer (2) transition into one another within the boundary portion.

16. The absorbent structure according to any one of the preceding claims, **characterized in that** the liquid distribution layer (4) includes a first and a second surface, wherein the first surface is in contact with the liquid storage layer (3), and wherein the liquid distribution layer (4) is compressed more strongly at its second surface than at its first surface.

17. A method for producing an absorbent structure, comprising at least the following steps:
- depositing a first layer, preferably an air-laid layer, which preferably includes at least cellulose fibers and bicomponent fibers, and/or a highly voluminous fleece layer from thermoplastic fibers for configuring a liquid absorption layer (2),
- depositing a second air-laid layer for configuring a liquid storage layer (3) which includes at least cellulose fibers and SAP-particles and/or SAP fibers,
- depositing a third air-laid layer for forming a liquid distribution layer (4),
- preferably applying a bonding agent layer, preferably a latex layer on one layer, preferably onto the layer structure thus obtained,
- preferably moving the layer structure through a heating device in order to bond the layer structure,
- supplying at least one layer, preferably of the layer structure to a calender including at least one smooth roller and an opposite roller, preferably with protrusions which form a calender gap,
- compressing the at least one layer, preferably of the layer structure in the calendar gap, wherein more than 20% of the SAP particles and/or SAP fibers arranged in the liquid storage layer (3) extend into the liquid distribution layer (4) from the liquid storage layer (3) and form a liquid-discharging contact between the liquid storage layer (3) and the liquid distribution layer (4).

18. Use of a device for producing an absorbent structure (1) according to any one of claims 1 to 16 and/or or for performing a method according to claim 17, said device comprising at least:
- a perforated band for depositing air-laid layers for forming a layer structure,
- a first air-laid forming device (10) from which at least cellulose fibers are pullable that form an air-laid layer,
- a second air-laid forming device (11) from which at least cellulose fibers and SAP particles and/or SAP fibers are depositable on the air-laid layer and preferably form a second air-laid layer,
- a depositing device for another layer;
- an application device through which a bonding agent (26), preferably a latex layer, is applicable;
- a heating device in which bicomponent fibers and/or the bonding agent (26) are activatable,
- a roller arrangement, preferably a calender, through which the layer structure is compressible,
- and a feed device (28) for SAP particles and/or SAP fibers which are supplied at least in a dosed manner and preferably also in a position-controlled manner and with partial penetration into the cellulose fibers of an air-laid layer, wherein the cellulose fibers are adjacent to cellulose fibers of the second air-laid forming device (11).

## Revendications

1. Structure absorbante avec une suite de couches comportant au moins une couche de réception du liquide (2), une couche de stockage du liquide (3) consécutive avec un polymère superabsorbant, de préférence des particules de polymère superabsorbant et/ou des fibres de polymère superabsorbant, et une couche de répartition du liquide (4) consécutive, les couches étant liées et formant une structure de couches, au moins la couche de stockage du liquide (3) et la couche de répartition du liquide (4) comportant chacune au moins un matériau airlaid, de préférence une couche airlaid contenant des fibres de cellulose comme composant principal, une zone du matériau airlaid comportant dans la couche de répartition du liquide (4) des pores plus étroites que dans une zone du matériau airlaid dans la couche de stockage du liquide (3), et la couche de stockage du liquide (3) comportant un polymère superabsorbant, en particulier des particules de polymère superabsorbant, qui en sortant de la couche de stockage du liquide (3) pénètre dans la couche de répartition du liquide (4) pour former un effet d'aspiration en retour pour le liquide qui est entré dans la couche de répartition du liquide (4) en passant par la couche de réception du liquide (2) et la couche de stockage du liquide (3), plus de 20 % des particules de polymère superabsorbant et/ou fibres de polymère superabsorbant, disposées dans la couche de stockage du liquide (3), pénétrant dans la couche de répartition du liquide (4).

2. Structure absorbante selon la revendication 1, **caractérisée en ce que** la couche de réception du liquide (2) comporte un matériau airlaid.

3. Structure absorbante selon la revendication 1 ou 2, **caractérisée en ce que** la couche de réception du liquide (2) comporte des fibres thermoplastiques.

4. Structure absorbante selon la revendication 3, **caractérisée en ce que** la couche de réception du liquide (2) comporte un non-tissé volumineux en fibres thermoplastiques.

5. Structure absorbante selon l'une quelconque des revendications précédentes, **caractérisée en ce que** le polymère superabsorbant, sous l'effet du refoulement des fibres de la couche de répartition du liquide (4), pénètre dans cette dernière en sortant de la couche de stockage du liquide (3).

6. Structure absorbante selon l'une quelconque des revendications précédentes 1 à 4, **caractérisée en ce que** le polymère superabsorbant, de préférence les particules de polymère superabsorbant, pénètre de manière irrégulière dans la couche de répartition du liquide (4) en sortant de la couche de stockage du liquide (3).

7. Structure absorbante selon l'une quelconque des revendications précédentes, **caractérisée en ce que** des particules de polymère superabsorbant et/ou des fibres de polymère superabsorbant pénètrent au moins sur un tiers de leur extension longitudinale dans la couche de stockage du liquide (3).

8. Structure absorbante selon l'une quelconque des revendications précédentes, **caractérisée en ce que** plus de 40 % des particules de polymère superabsorbant et/ou des fibres de polymère superabsorbant, disposées dans la couche de stockage du liquide (3), pénètrent dans la couche de répartition du liquide (4).

9. Structure absorbante selon l'une quelconque des revendications précédentes, **caractérisée en ce que** plus de 40 %, mais moins de 70 % des particules de polymère superabsorbant et/ou des fibres de polymère superabsorbant, disposées dans la couche de stockage du liquide (3), pénètrent dans la couche de répartition du liquide (4).

10. Structure absorbante selon l'une quelconque des revendications précédentes, **caractérisée en ce que** le matériau en polymère superabsorbant, pénétrant dans la couche de répartition du liquide (4), s'est agrandi après l'absorption,de liquide davantage dans la couche de stockage du liquide (3) que dans la couche de répartition du liquide (4).

11. Structure absorbante selon l'une quelconque des revendications précédentes, **caractérisée en ce que** les particules de polymère superabsorbant et/ ou les fibres de polymère superabsorbant, pénétrant dans la couche de répartition du liquide (4), se sont retirées au moins partiellement dans la couche de répartition du liquide (4), après l'absorption du liquide.

12. Structure absorbante selon l'une quelconque des revendications précédentes, **caractérisée en ce que** la structure de couches à l'intérieur de la couche de répartition du liquide (4) et/ou de la couche de stockage du liquide (3) et/ou de la couche de réception du liquide (2) et/ou dans une zone limite entre la couche de répartition du liquide (4) et/ou la couche de stockage du liquide (3) et/ou la couche de réception du liquide (2) comporte des cavités.

13. Structure absorbante selon l'une quelconque des revendications précédentes, **caractérisée en ce que** les cavités réalisées dans la couche de stockage du liquide (3) sont plus grandes que celles dans la couche de réception du liquide (2) et/ou dans la couche de répartition du liquide (4).

14. Structure absorbante selon l'une quelconque des revendications précédentes, **caractérisée en ce qu'**au moins une partie des fibres de cellulose des couches airlaid dans la couche de répartition du liquide (4) et/ou la couche de stockage du liquide (3) et/ou la couche de réception du liquide (2) sont mélangées entre elles dans une zone limite située respectivement entre celles-ci.

15. Structure absorbante selon l'une quelconque des revendications précédentes, **caractérisée en ce que** les couches airlaid de la couche de répartition du liquide (4) et/ou de la couche de stockage du liquide (3) et/ou de la couche de réception du liquide (2) se prolongent les unes dans les autres à l'intérieur de la zone limite.

16. Structure absorbante selon l'une quelconque des revendications précédentes, **caractérisée en ce que** la couche de répartition du liquide (4) comporte une première et une deuxième surface, la première surface étant en contact avec la couche de stockage du liquide (3), et la couche de répartition du liquide (4) étant plus fortement compactée sur sa deuxième surface que sur sa première surface.

17. Procédé permettant la fabrication d'une structure absorbante (1), comportant au moins les étapes suivantes :
- pose d'une première couche, de préférence une couche airlaid, qui comporte de préférence au moins des fibres de cellulose et des fibres bicomposantes, et/ou d'une couche de non-tissé très volumineux en fibres thermoplastiques, pour la formation de la couche de réception du liquide (2),
- pose d'une deuxième ouche airlaid pour la formation d'une couche de stockage du liquide (3), qui comporte au moins des fibres de cellulose et des particules de polymère superabsorbant et/ou des fibres de polymère superabsorbant,
- pose d'une troisième couche airlaid pour la formation d'une couche de répartition du liquide (4),
- de préférence application d'une couche de liants, de préférence une couche de latex sur une couche, de préférence sur la structure de couches ainsi obtenue,
- de préférence passage de la structure de couches à travers un moyen de chauffage pour lier la structure de couches,
- acheminement d'au moins une couche, de préférence la structure de couches vers une calandre, comportant au moins un rouleau de lissage et un contre-rouleau muni, de préférence, de reliefs qui forment une fente de calandrage,
- compactage de ladite au moins une couche, de préférence la structure de couches dans la fente de calandrage, au cours duquel processus au moins 20 % des particules de polymère superabsorbant et/ou des fibres de polymère superabsorbant, disposées dans la couche de stockage du liquide (3), pénètrent dans la couche de répartition du liquide (4) en sortant de la couche de stockage du liquide (3) et forment entre la couche de stockage du liquide (3) et la couche de répartition du liquide (4) un contact refoulant le liquide.

18. Utilisation d'un dispositif permettant la fabrication d'une structure absorbante (1) selon l'une quelconque des revendications 1 à 16 et/ou permettant la mise en oeuvre d'un procédé selon la revendication 17, comportant au moins :
- une bande tamis pour déposer les couches airlaid pour la formation d'une structure de couches,
- un premier dispositif de formage airlaid (10), par lequel au moins des fibres de cellulose peuvent être retirées, lesquelles forment une couche airlaid,
- un deuxième dispositif de formage airlaid (11), par lequel au moins des fibres de cellulose et des particules de polymère superabsorbant et/ou des fibres de polymère superabsorbant peuvent être déposées sur la couche airlaid et forment de préférence une deuxième couche airlaid,
- un dispositif de pose pour une couche supplémentaire ;
- un moyen d'application, par lequel un liant (26), de préférence une couche de latex, peut être appliqué ,
- un moyen de chauffage, dans lequel les fibres bicomposantes et/ou le liant (26) sont activables,
- un ensemble de rouleaux, de préférence une calandre, qui permet de compacter la structure de couches,
- et un dispositif d'acheminement (28) des particules de polymère superabsorbant et/ou des fibres de polymère superabsorbant qui, au moins dosées et de préférence aussi contrôlées en position, sont acheminées vers une couche airlaid moyennant une pénétration partielle dans les fibres de cellulose, qui sont adjacentes aux fibres de cellulose du deuxième dispositif de formage airlaid (11).
